(19) European Patent Office / Europäisches Patentamt / Office européen des brevets

(11) **EP 4 755 403 A1**

(12) **EUROPEAN PATENT APPLICATION**
published in accordance with Art. 153(4) EPC


(43) Date of publication:
**10.06.2026 Bulletin 2026/24**

(21) Application number: **24850930.9**

(22) Date of filing: **02.08.2024**

(51) International Patent Classification (IPC):
***A61K 47/68*** (2017.01) ***A61K 47/65*** (2017.01)
***A61K 31/506*** (2006.01) ***C07D 491/22*** (2006.01)
***A61P 35/00*** (2006.01) ***A61P 37/02*** (2006.01)

(52) Cooperative Patent Classification (CPC):
**A61K 31/506; A61K 47/65; A61K 47/68; A61P 35/00; A61P 37/02; C07D 491/22**

(86) International application number:
**PCT/CN2024/109472**

(87) International publication number:
**WO 2025/031285 (13.02.2025 Gazette 2025/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **04.08.2023 CN 202310976626**
**08.08.2023 CN 202310990738**
**09.11.2023 CN 202311485462**



(71) Applicants:
- **Jiangsu Hengrui Pharmaceuticals Co., Ltd.**
  **Lianyungang, Jiangsu 222047 (CN)**
- **Shanghai Hengrui Pharmaceutical Co., Ltd.**
  **Shanghai 200245 (CN)**

(72) Inventors:
- **LIN, Guang**
  **Shanghai 200245 (CN)**
- **ZHANG, Haoying**
  **Shanghai 200245 (CN)**
- **YE, Xin**
  **Shanghai 200245 (CN)**
- **YOU, Lingfeng**
  **Shanghai 200245 (CN)**
- **HE, Feng**
  **Shanghai 200245 (CN)**

(74) Representative: **Potter Clarkson**
**Chapel Quarter**
**Mount Street**
**Nottingham NG1 6HQ (GB)**


Remarks:
The complete document including Reference Table(s) and the Sequence Listing(s) can be downloaded from the EPO website

(54) **ANTI-PSMA ANTIBODY-DRUG CONJUGATE AND PHARMACEUTICAL USE THEREOF**


(57) An anti-PSMA antibody-drug conjugate and the pharmaceutical use thereof. Specifically, disclosed is an anti-PSMA antibody-drug conjugate as represented by the general formula Pc-L-Y-D, wherein Pc is an anti-PSMA antibody or an antigen-binding fragment thereof, and L, Y and n are as defined in the description.


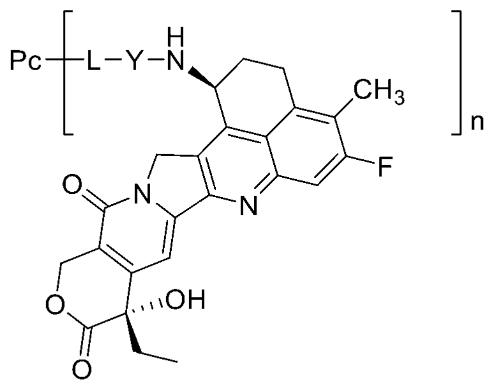

Pc-L-Y-D

EP 4 755 403 A1

## Description

The present disclosure claims priority to CN202310976626.5 filed on Aug. 4, 2023, CN202310990738.6 filed on Aug. 8, 2023, and CN202311485462.2 filed on Nov. 9, 2023.

## TECHNICAL FIELD

The present disclosure relates to an anti-PSMA antibody-drug conjugate (especially an anti-PSMA antibody-exatecan analog conjugate), a preparation method therefor, a pharmaceutical composition comprising same, and use thereof in the manufacture of a medicament for treating a PSMA-mediated disease or disorder, particularly in the manufacture of a medicament for treating and/or preventing a tumor or cancer.

## BACKGROUND

The statements herein merely provide background information related to the present disclosure and may not necessarily constitute the prior art.

Statistics from the American Cancer Society in 2019 showed that prostate cancer ranked first among new cancer cases in men in the United States, with 174,650 cases (CA CANCER J CLIN 2019; 69: 7-34). Surgery and radiotherapy are usually selected for clinically localized prostate cancer, and surgical or chemical androgen deprivation therapy (ADT) is usually the first treatment for locally advanced or metastatic prostate cancer. Sipuleucel-T cell immunotherapy can be selected for the early stage of castration-resistant prostate cancer (CRPC), and drug therapies such as androgen inhibitors, androgen receptor antagonists, radiotherapy drugs for bone metastasis, and chemotherapeutic drugs acting on microtubules can be selected for patients with metastatic castration-resistant prostate cancer (mCRPC) as appropriate. However, each of the therapies can only prolong the survival by a few months, so it is necessary to seek effective therapies (European Urology, 66(6), 1190-1193; Journal of Nuclear Medicine, 59(2), 177-182).

A prostate specific membrane antigen (PSMA) belongs to glutamate carboxypeptidase II (GCPII). PSMA consists of 750 amino acids, including an intracellular domain (containing 19 amino acids), a transmembrane domain (containing 24 amino acids), and an extracellular domain (containing 707 amino acids). The crystal structure shows that the extracellular part consists of 3 domains, namely a protease-like domain, an apical domain, and a C-terminal domain (The EMBO Journal (2006) 25, 1375-1384). In the prostate, PSMA is expressed on epithelial cells. In addition, it can also be expressed by non-prostate tissues, such as the small intestine, proximal renal tubules, and salivary glands, but at levels much lower than in the prostate tissue. PSMA is highly expressed in prostate cancer cells, particularly in metastatic diseases, hormone refractory diseases, and high-grade lesions. In addition, PSMA is also highly expressed in endothelial cells of neovasculature of all solid tumors, but not in normal vasculature, so it is a target for the treatment of solid tumors (Clinical Cancer Research, Vol. 3, 81-85, January 1997; Urologic Oncology: Seminars and Original Investigations, 1(1), 18-28; Clin Cancer Res., 2010 November 15; 16(22): 5414-5423). The androgen deprivation therapy and androgen receptor antagonist therapy can both up-regulate the expression of PSMA in the prostate (J Nucl Med, 2017; 58: 81-84), which provides a basis for targeted therapy in combination with traditional hormone therapy.

An antibody-drug conjugate (ADC) links a monoclonal antibody or an antibody fragment to a biologically active drug via a linker, fully exploiting the binding specificity of the antibody to surface antigens of normal cells and tumor cells and the high efficiency of the drug (such as cytotoxic agent), and also avoiding defects of the poor therapeutic effect of the antibody and excessive toxic and side effects of the drug. The antibody-drug conjugate can kill tumor cells more precisely and has a reduced effect on normal cells compared to conventional chemotherapeutic drugs in the past.

A variety of ADC drugs have been used in clinical or clinical studies, such as Kadcyla, which is an ADC drug formed by Her2-targeted Trastuzumab and DM1. Meanwhile, there are also PSMA-targeted ADC drugs for clinical therapeutic studies. ARX-517, developed by Ambrx, is currently in phase I clinical stage. Due to poor efficacy, PSMA-ADC from Cytogen was discontinued in phase II clinical stage, and MEDI-3726 from MedImmune and MLN-2704 from BZL Biologics Inc. were discontinued at the end of phase I clinical stage. The development of new ADC drugs by adopting different strategies has wide prospects.

## SUMMARY

The present disclosure relates to an antibody-drug conjugate in which an anti-PSMA antibody or an antigen-binding fragment is conjugated to a cytotoxic substance (e.g., an exatecan analog).

The present disclosure provides an antibody-drug conjugate of general formula Pc-L-Y-D or a pharmaceutically acceptable salt thereof:

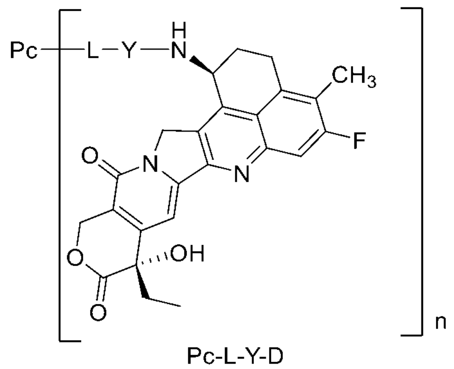

Pc-L-Y-D

wherein:

Pc is an anti-PSMA antibody or an antigen-binding fragment thereof, comprising a heavy chain variable region and a light chain variable region, wherein:

the anti-PSMA antibody comprises 1) a HCDR1, a HCDR2, and a HCDR3 comprised in a heavy chain variable region set forth in SEQ ID NO: 3; and 2) a LCDR1, a LCDR2, and a LCDR3 comprised in a light chain variable region set forth in SEQ ID NO: 4;

preferably, the anti-PSMA antibody comprises a heavy chain variable region and a light chain variable region, wherein:

a. the heavy chain variable region comprises a HCDR1, a HCDR2, and a HCDR3 set forth in SEQ ID NO: 5, SEQ ID NO: 6, and SEQ ID NO: 7, respectively; the light chain variable region comprises a LCDR1, a LCDR2, and a LCDR3 set forth in SEQ ID NO: 8, SEQ ID NO: 9, and SEQ ID NO: 10, respectively, wherein the CDR regions are determined according to the Kabat numbering scheme; or

b. the heavy chain variable region comprises a HCDR1, a HCDR2, and a HCDR3 set forth in SEQ ID NO: 26, SEQ ID NO: 27, and SEQ ID NO: 28, respectively; the light chain variable region comprises a LCDR1, a LCDR2, and a LCDR3 set forth in SEQ ID NO: 29, SEQ ID NO: 30, and SEQ ID NO: 10, respectively, wherein the CDR regions are determined according to the IMGT numbering scheme; or

c. the heavy chain variable region comprises a HCDR1, a HCDR2, and a HCDR3 set forth in SEQ ID NO: 31, SEQ ID NO: 32, and SEQ ID NO: 7, respectively; the light chain variable region comprises a LCDR1, a LCDR2, and a LCDR3 set forth in SEQ ID NO: 8, SEQ ID NO: 9, and SEQ ID NO: 10, respectively, wherein the CDR regions are determined according to the Chothia numbering scheme;

Y is -O-C$R^1R^2$-C(O)-, wherein $R^1$ is $C_{1-6}$ haloalkyl or $C_{3-6}$ cycloalkyl; $R^2$ is selected from the group consisting of a hydrogen atom, $C_{1-6}$ haloalkyl, and $C_{3-6}$ cycloalkyl; or, $R^1$ and $R^2$, together with the carbon atom to which they are attached, form $C_{3-6}$ cycloalkyl;

L is a linker unit;

n is a decimal or an integer from 1 to 10.

The present disclosure provides an antibody-drug conjugate of general formula Pc-L-Y-D or a pharmaceutically acceptable salt thereof:

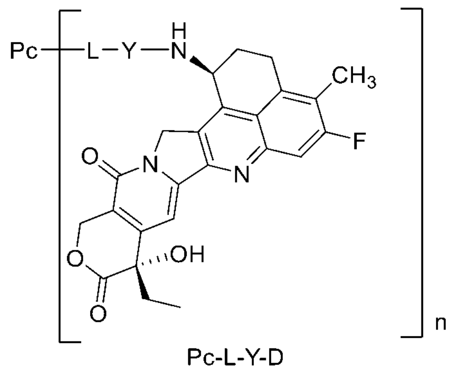

Pc-L-Y-D

wherein:

Pc is an anti-PSMA antibody or an antigen-binding fragment thereof, comprising a heavy chain variable region and a light chain variable region, wherein:

the heavy chain variable region comprises the HCDR1, the HCDR2, and the HCDR3 set forth in SEQ ID NO: 5, SEQ ID NO: 6, and SEQ ID NO: 7, respectively; the light chain variable region comprises the LCDR1, the LCDR2, and the LCDR3 set forth in SEQ ID NO: 8, SEQ ID NO: 9, and SEQ ID NO: 10, respectively;

Y is $-O-CR^1R^2-C(O)-$, wherein $R^1$ is $C_{1-6}$ haloalkyl or $C_{3-6}$ cycloalkyl; $R^2$ is selected from the group consisting of a hydrogen atom, $C_{1-6}$ haloalkyl, and $C_{3-6}$ cycloalkyl; or, $R^1$ and $R^2$, together with the carbon atom to which they are attached, form $C_{3-6}$ cycloalkyl;

L is a linker unit;

n is a decimal or an integer from 1 to 10.

In some embodiments, in the antibody-drug conjugate of general formula Pc-L-Y-D or the pharmaceutically acceptable salt thereof as described above, the anti-PSMA antibody is a mouse antibody, a chimeric antibody, a humanized antibody, or a human antibody. In some embodiments, in the antibody-drug conjugate of general formula Pc-L-Y-D or the pharmaceutically acceptable salt thereof as described above, the anti-PSMA antibody is a humanized antibody.

In some embodiments, in the antibody-drug conjugate of general formula Pc-L-Y-D or the pharmaceutically acceptable salt thereof according to any one of the above, the anti-PSMA antibody or the antigen-binding fragment thereof comprises a heavy chain variable region set forth in SEQ ID NO: 11 or SEQ ID NO: 12, and a light chain variable region set forth in SEQ ID NO: 13 or SEQ ID NO: 14; preferably, the anti-PSMA antibody or the antigen-binding fragment thereof comprises a heavy chain variable region set forth in SEQ ID NO: 12 and a light chain variable region set forth in SEQ ID NO: 14.

In some embodiments, in the antibody-drug conjugate of general formula Pc-L-Y-D or the pharmaceutically acceptable salt thereof according to any one of the above, the anti-PSMA antibody comprises a heavy chain constant region and a light chain constant region; preferably, the heavy chain constant region is selected from the group consisting of human IgG1, IgG2, IgG3, and IgG4 constant regions and conventional variants thereof, and the light chain constant region is selected from the group consisting of human antibody κ and λ chain constant regions and conventional variants thereof; more preferably, the heavy chain constant region is set forth in SEQ ID NO: 15 or SEQ ID NO: 16, and the light chain constant region is set forth in SEQ ID NO: 17 or SEQ ID NO: 18; most preferably, the heavy chain constant region is set forth in SEQ ID NO: 15, and the light chain constant region is set forth in SEQ ID NO: 17.

In some embodiments, in the antibody-drug conjugate of general formula Pc-L-Y-D or the pharmaceutically acceptable salt thereof according to any one of the above, the anti-PSMA antibody comprises a heavy chain set forth in SEQ ID NO: 19 and a light chain set forth in SEQ ID NO: 20.

In some embodiments, in the antibody-drug conjugate of general formula Pc-L-Y-D or the pharmaceutically acceptable salt thereof according to any one of the above, n is a decimal or an integer from 1 to 8, preferably from 3 to 8, and more preferably from 5 to 8.

In some embodiments, in the antibody-drug conjugate of general formula Pc-L-Y-D or the pharmaceutically acceptable salt thereof according to any one of the above, Y is selected from the group consisting of:

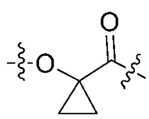, 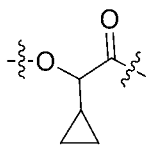, 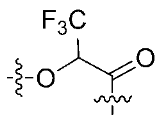, 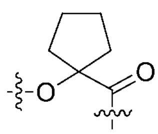, and 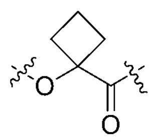;

wherein the O-terminus of Y is attached to L.

In some embodiments, the antibody-drug conjugate of general formula Pc-L-Y-D or the pharmaceutically acceptable salt thereof according to any one of the above is an antibody-drug conjugate of general formula Pc-L-D1 below or a pharmaceutically acceptable salt thereof:

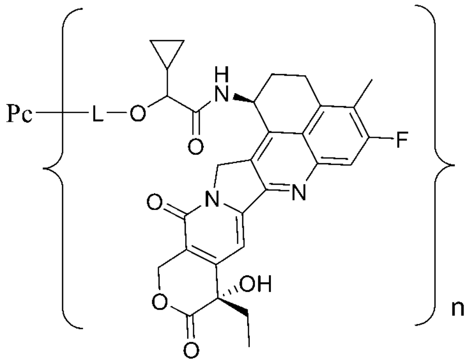

Pc-L-D1;

wherein Pc, n, and L are as defined above.

In some embodiments, the antibody-drug conjugate of general formula Pc-L-Y-D or the pharmaceutically acceptable salt thereof according to any one of the above is an antibody-drug conjugate of general formula Pc-L-D2 below or a pharmaceutically acceptable salt thereof:

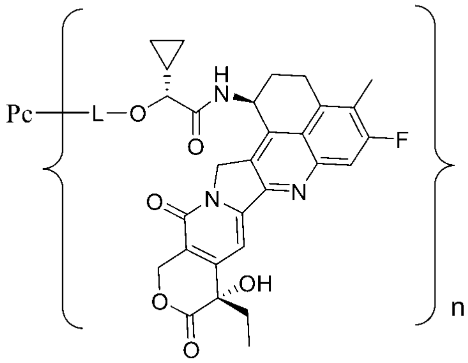

Pc-L-D2;

wherein Pc, n, and L are as defined above.

In some embodiments, in the antibody-drug conjugate of general formula Pc-L-Y-D or the pharmaceutically acceptable salt thereof according to any one of the above, the linker unit -L- is $-L^1-L^2-L^3-$, wherein

$L^1$ is selected from the group consisting of -(succinimid-3-yl-*N*)-W-C(O)- and -C(O)-W-C(O)-, wherein W is selected from the group consisting of $C_{1-6}$ alkyl and $C_{1-6}$ alkyl-$C_{3-6}$ cycloalkyl;

$L^2$ is a peptide residue consisting of 2 to 7 amino acid residues, wherein the amino acid residues are selected from the group consisting of amino acid residues formed from amino acids of phenylalanine, glycine, valine, lysine, citrulline, serine, glutamic acid, and aspartic acid;

$L^3$ is selected from the group consisting of $-NR^3(CR^4R^5)_t-$, $-C(O)NR^3$, $-C(O)NR^3(CH_2)_t-$, and a chemical bond, wherein t is an integer from 1 to 6;

$R^3$, $R^4$, and $R^5$ are identical or different and are each independently selected from the group consisting of a hydrogen atom, halogen, $C_{1-6}$ alkyl, $C_{1-6}$ haloalkyl, $C_{1-6}$ deuterated alkyl, and $C_{1-6}$ hydroxyalkyl;

wherein the $L^1$ terminus is attached to Pc, and the $L^3$ terminus is attached to a drug.

In some embodiments, in the antibody-drug conjugate of general formula Pc-L-Y-D or the pharmaceutically acceptable salt thereof according to any one of the above, the linker unit -L- is $-L^1-L^2-L^3-$, wherein

$L^1$ is

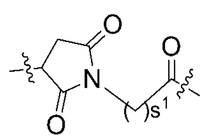

,

and $s^1$ is an integer from 2 to 8;

$L^2$ is a tetrapeptide residue; preferably, $L^2$ is a tetrapeptide residue of GGFG (SEQ ID NO: 25);

$L^3$ is $-NR^3(CR^4R^5)_t-$, wherein $R^3$, $R^4$, and $R^5$ are identical or different and are each independently a hydrogen atom or $C_{1-6}$ alkyl, and t is 1 or 2;

wherein the $L^1$ terminus is attached to Pc, and the $L^3$ terminus is attached to a drug.

In some embodiments, in the antibody-drug conjugate of general formula Pc-L-Y-D or the pharmaceutically acceptable salt thereof according to any one of the above, the antibody-drug conjugate is:

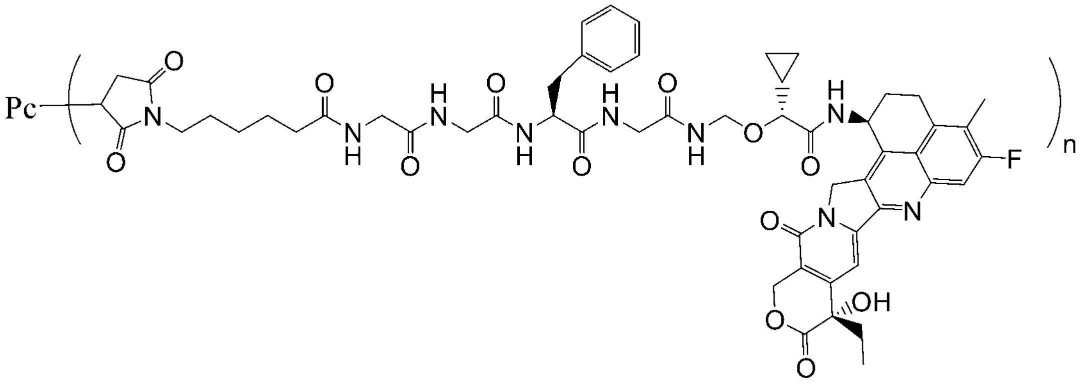

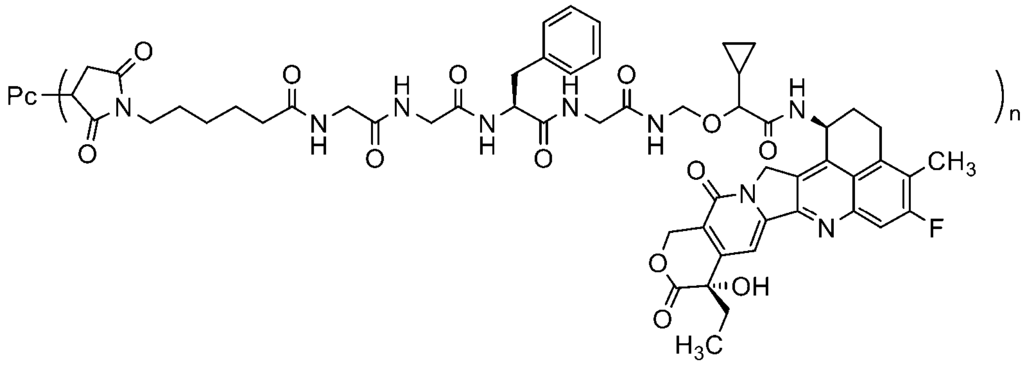

,

or

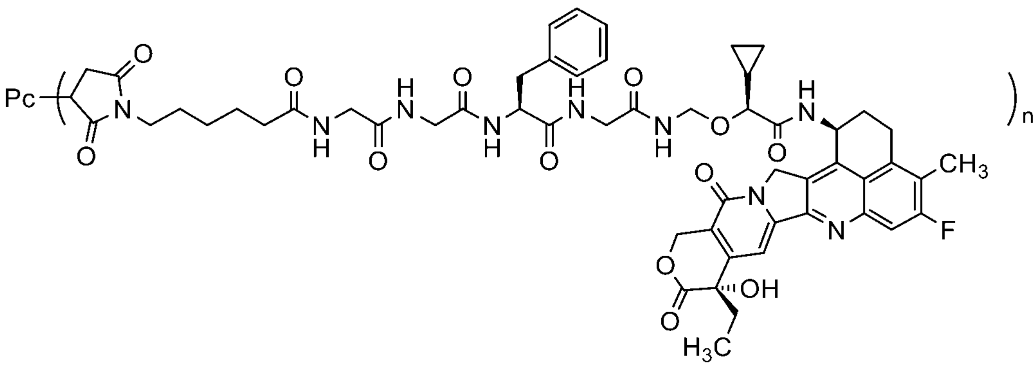

,

wherein:

Pc is an anti-PSMA antibody or an antigen-binding fragment thereof, comprising a heavy chain variable region and a light chain variable region, wherein:

the heavy chain variable region comprises the HCDR1, the HCDR2, and the HCDR3 set forth in SEQ ID NO: 5, SEQ ID NO: 6, and SEQ ID NO: 7, respectively; the light chain variable region comprises the LCDR1, the LCDR2, and the LCDR3 set forth in SEQ ID NO: 8, SEQ ID NO: 9, and SEQ ID NO: 10, respectively;

preferably, the anti-PSMA antibody or the antigen-binding fragment thereof comprises a heavy chain variable region set forth in SEQ ID NO: 11 or SEQ ID NO: 12, and a light chain variable region set forth in SEQ ID NO: 13 or SEQ ID NO: 14;
more preferably, the anti-PSMA antibody or the antigen-binding fragment thereof comprises a heavy chain variable region set forth in SEQ ID NO: 12 and a light chain variable region set forth in SEQ ID NO: 14;
most preferably, the anti-PSMA antibody comprises a heavy chain set forth in SEQ ID NO: 19 and a light chain set forth in SEQ ID NO: 20;
n is a decimal or an integer from 1 to 10.

In some embodiments, in the antibody-drug conjugate of general formula Pc-L-Y-D or the pharmaceutically acceptable salt thereof according to any one of the above, the antibody-drug conjugate is:

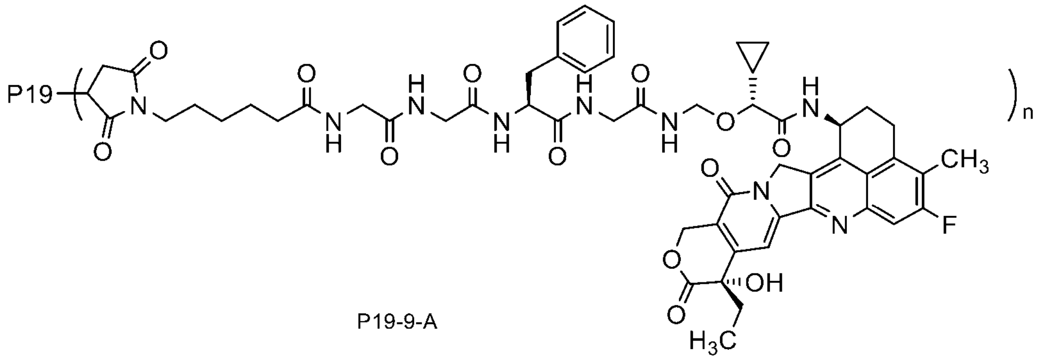

P19-9-A

wherein:

P19 is an anti-PSMA antibody, comprising the heavy chain set forth in SEQ ID NO: 19 and the light chain set forth in SEQ ID NO: 20;
n is a decimal or an integer from 1 to 8, preferably from 3 to 8, and more preferably from 5 to 8.

The present disclosure further provides a method for preparing an antibody-drug conjugate of general formula Pc-9-A, which comprises the following step:

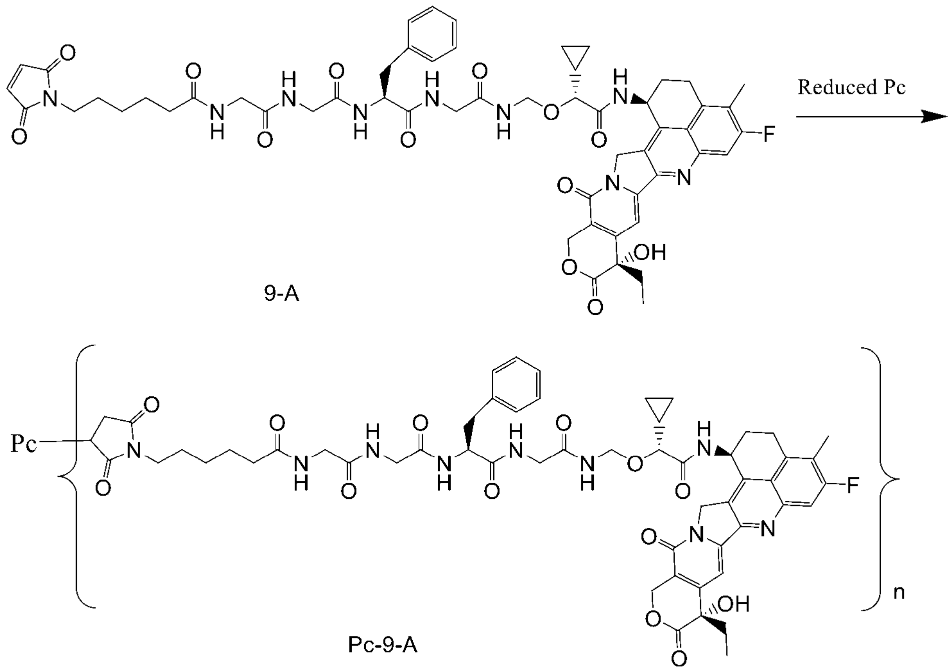


9-A

Pc-9-A

In the reaction described above, compound 9-A and the reduced Pc are subjected to a coupling reaction to give the compound of general formula (Pc-9-A), wherein:

Pc is the anti-PSMA antibody or the antigen-binding fragment thereof as described above, comprising a heavy chain variable region and a light chain variable region, wherein:

the heavy chain variable region comprises the HCDR1, the HCDR2, and the HCDR3 set forth in SEQ ID NO: 5, SEQ ID NO: 6, and SEQ ID NO: 7, respectively; the light chain variable region comprises the LCDR1, the LCDR2, and the LCDR3 set forth in SEQ ID NO: 8, SEQ ID NO: 9, and SEQ ID NO: 10, respectively;
preferably, the anti-PSMA antibody or the antigen-binding fragment thereof comprises a heavy chain variable region set forth in SEQ ID NO: 11 or SEQ ID NO: 12, and a light chain variable region set forth in SEQ ID NO: 13 or SEQ ID NO: 14;
more preferably, the anti-PSMA antibody or the antigen-binding fragment thereof comprises a heavy chain variable region set forth in SEQ ID NO: 12 and a light chain variable region set forth in SEQ ID NO: 14;
most preferably, the anti-PSMA antibody or the antigen-binding fragment thereof comprises a heavy chain with the sequence set forth in SEQ ID NO: 19 and a light chain with the sequence set forth in SEQ ID NO: 20;
n is as defined in general formula Pc-L-Y-D.

The present disclosure further provides a method for preparing an antibody-drug conjugate of P19-9-A, which comprises the following step:

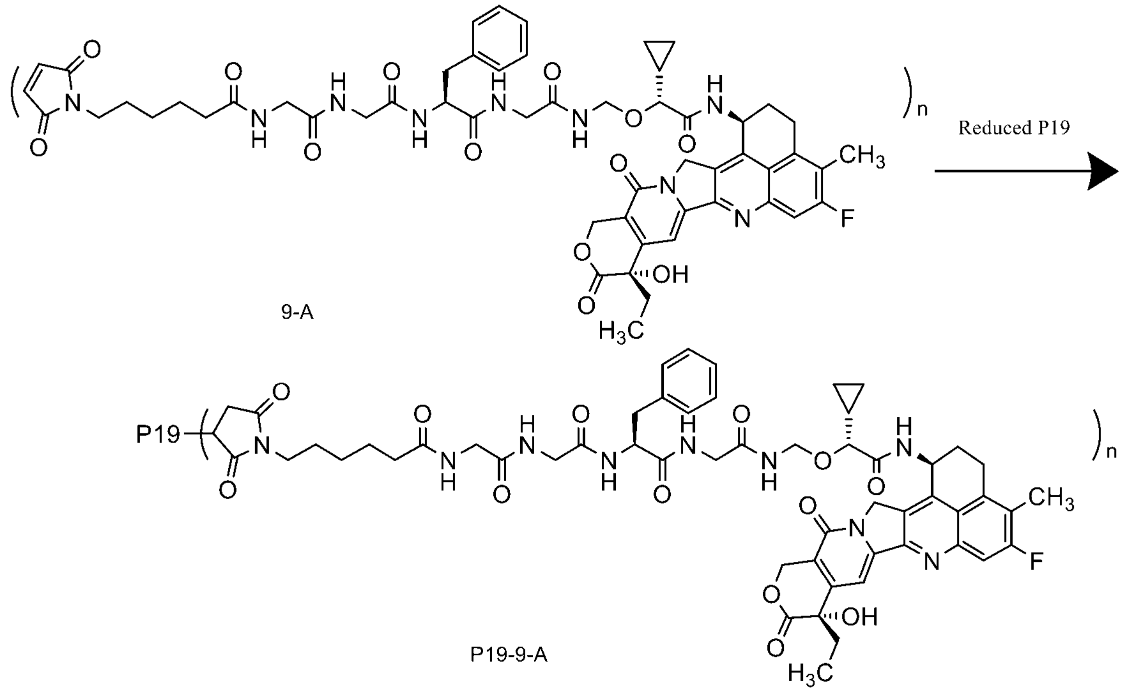


9-A

P19-9-A

subjecting compound 9-A and the reduced P19 to a coupling reaction to give the compound of formula P19-9-A, wherein:

n is a decimal or an integer from 1 to 8, preferably from 3 to 8, and more preferably from 5 to 8;
P19 is an anti-PSMA antibody, comprising a heavy chain with the sequence set forth in SEQ ID NO: 19 and a light chain with the sequence set forth in SEQ ID NO: 20.

In some embodiments, n represents the drug loading of the antibody-drug conjugate, and may also be referred to as a DAR value, which may be determined by conventional methods such as UV/visible spectroscopy, mass spectrometry, an ELISA assay, and HPLC. In some embodiments, n is an average value of 0 to 10, preferably 1 to 10, and more preferably 1 to 8, or 2 to 8, or 2 to 7, or 2 to 4, or 3 to 8, or 4 to 8, or 5 to 8, or 3 to 7, or 3 to 6, or 4 to 7, or 4 to 6, or 4 to 5.
In some embodiments, n is an average value of 1, 2, 3, 4, 5, 6, 7, 8, 9, and 10.
In another aspect, the present disclosure provides a pharmaceutical composition comprising the antibody-drug conjugate or the pharmaceutically acceptable salt thereof according to the present disclosure, and one or more pharmaceutically acceptable excipients, diluents, or carriers.
In another aspect, the present disclosure provides use of the antibody-drug conjugate or the pharmaceutically acceptable salt thereof, or the pharmaceutical composition comprising same according to the present disclosure as a medicament, preferably as a medicament for treating or preventing a tumor or cancer.

In another aspect, the present disclosure provides use of the antibody-drug conjugate or the pharmaceutically acceptable salt thereof, or the pharmaceutical composition comprising same according to the present disclosure in the manufacture of a medicament for treating a PSMA-mediated disease or disorder, wherein the PSMA-mediated disease or disorder is preferably a cancer associated with high, moderate, or low expression of PSMA.

In another aspect, the present disclosure provides use of the antibody-drug conjugate or the pharmaceutically acceptable salt thereof, or the pharmaceutical composition comprising same according to the present disclosure in the manufacture of a medicament for treating or preventing a tumor or cancer, wherein the tumor or cancer is preferably prostate cancer, head and neck squamous cell carcinoma, head and neck cancer, brain cancer, neuroglioma, glioblastoma multiforme, neuroblastoma, neuroendocrine neoplasm, throat cancer, nasopharyngeal carcinoma, esophageal cancer, thyroid cancer, malignant pleural mesothelioma, lung cancer, breast cancer, liver cancer, hepatobiliary cancer, pancreatic cancer, gastric cancer, colorectal cancer, renal cancer, ovarian cancer, endometrial cancer, cervical cancer, bladder cancer, testicular cancer, melanoma, leukemia, lymphoma, chondrosarcoma, multiple myeloma, myelodysplastic syndrome, Krukenberg tumor, squamous cell carcinoma, Ewing sarcoma, urothelial carcinoma, or Merkel cell carcinoma, preferably prostate cancer; more preferably, the lymphoma is selected from the group consisting of Hodgkin lymphoma, non-Hodgkin lymphoma, diffuse large B-cell lymphoma, follicular lymphoma, primary mediastinal large B-cell lymphoma, mantle cell lymphoma, small lymphocytic lymphoma, T-cell/histiocyte-rich large B-cell lymphoma, and lymphoplasmacytic lymphoma, the lung cancer is selected from the group consisting of non-small cell lung cancer and small cell lung cancer, and the leukemia is selected from the group consisting of chronic myeloid leukemia, acute myeloid leukemia, lymphocytic leukemia, lymphoblastic leukemia, acute lymphoblastic leukemia, chronic lymphocytic leukemia, and myeloid cell leukemia.

In another aspect, the present disclosure further relates to a method for treating and/or preventing a tumor or cancer, which comprises administering to a subject in need thereof a therapeutically effective dose of the antibody-drug conjugate or the pharmaceutically acceptable salt thereof, or the pharmaceutical composition comprising same according to the present disclosure, wherein the tumor or cancer is preferably a cancer associated with high, moderate, or low expression of PSMA.

In another aspect, the present disclosure further relates to a method for treating or preventing a tumor or cancer, which comprises administering to a subject in need thereof a prophylactically or therapeutically effective dose of the antibody-drug conjugate or the pharmaceutically acceptable salt thereof, or the pharmaceutical composition comprising same according to the present disclosure, wherein the tumor or cancer is preferably prostate cancer, head and neck squamous cell carcinoma, head and neck cancer, brain cancer, neuroglioma, glioblastoma multiforme, neuroblastoma, neuroendocrine neoplasm, throat cancer, nasopharyngeal carcinoma, esophageal cancer, thyroid cancer, malignant pleural mesothelioma, lung cancer, breast cancer, liver cancer, hepatobiliary cancer, pancreatic cancer, gastric cancer, colorectal cancer, renal cancer, ovarian cancer, endometrial cancer, cervical cancer, bladder cancer, testicular cancer, melanoma, leukemia, lymphoma, chondrosarcoma, multiple myeloma, myelodysplastic syndrome, Krukenberg tumor, squamous cell carcinoma, Ewing sarcoma, urothelial carcinoma, or Merkel cell carcinoma, preferably prostate cancer; more preferably, the lymphoma is selected from the group consisting of Hodgkin lymphoma, non-Hodgkin lymphoma, diffuse large B-cell lymphoma, follicular lymphoma, primary mediastinal large B-cell lymphoma, mantle cell lymphoma, small lymphocytic lymphoma, T-cell/histiocyte-rich large B-cell lymphoma, and lymphoplasmacytic lymphoma, the lung cancer is selected from the group consisting of non-small cell lung cancer and small cell lung cancer, and the leukemia is selected from the group consisting of chronic myeloid leukemia, acute myeloid leukemia, lymphocytic leukemia, lymphoblastic leukemia, acute lymphoblastic leukemia, chronic lymphocytic leukemia, and myeloid cell leukemia.

In another aspect, the present disclosure further provides the anti-PSMA antibody or the antibody-drug conjugate thereof described above as a medicament, preferably as a medicament for treating or preventing a tumor or cancer, and more preferably as a medicament for treating a PSMA-mediated disease or disorder; wherein the PSMA-mediated disease or disorder is preferably a cancer associated with high, moderate, or low expression of PSMA.

The active compound (e.g., the antibody-drug conjugate or the pharmaceutically acceptable salt thereof according to the present disclosure) may be formulated in a form suitable for administration by any suitable route, preferably in a form of a unit dose, or in a form of a unit dose that can be self-administered by a subject. The unit dose of the active compound or composition of the present disclosure may be in the form of a tablet, a capsule, a cachet, a vial, a powder, a granule, a lozenge, a suppository, a freeze-dried powder, or a liquid formulation.

The administration dose of the active compound or composition used in the treatment method of the present disclosure will generally vary with the severity of the disease, the weight of the subject, and the relative efficacy of the active compound. As a general guide, a suitable unit dose may be 0.1 mg to 1000 mg.

The pharmaceutical composition of the present disclosure may comprise, in addition to the active compound, one or more auxiliary materials selected from the group consisting of a filler, a diluent, a binder, a wetting agent, a disintegrant, an excipient, and the like. Depending on the method of administration, the composition may comprise 0.1-99 wt.% of the active compound.

In another aspect, the present disclosure provides a kit comprising materials useful for treating or preventing the

disease described above. The kit comprises a container and a label or package insert. Suitable containers include, for example, bottles, vials, syringes, and IV solution bags. The container may be formed from a variety of materials such as glass or plastic. The container contains the antibody-drug conjugate or the pharmaceutically acceptable salt thereof according to the present disclosure alone or in combination with another composition. As an example, the kit may comprise: (a) a first container containing the antibody-drug conjugate; and (b) a second container containing an additional composition. Additionally, the kit may further comprise a second (or third) container containing a pharmaceutically acceptable buffer. The kit may further comprise other required materials, including other buffers, diluents, filters, needles, and syringes. The PSMA antibody and the antibody-drug conjugate provided by the present disclosure have good affinity for cell surface antigens, good endocytosis efficiency and strong tumor inhibition efficiency, have broader drug application window, higher tumor inhibition effect and therapeutic activity, and better safety, pharmacokinetic properties and druggability (such as stability), and are more suitable for clinical drug application.

## BRIEF DESCRIPTION OF THE DRAWINGS

FIG. 1A shows the binding activity of the antibody to LNCaP cells.
FIG. 1B shows the binding activity of the antibody to 22RV1 cells.
FIG. 1C shows the binding activity of the antibody to PC-3 cells.
FIG. 2 shows PK of ADC molecules in SD rats.
FIG. 3 shows PK of ADC molecules in cynomolgus monkeys.

## DETAILED DESCRIPTION

To facilitate the understanding of the present disclosure, certain technical and scientific terms are described below. Unless otherwise specifically defined herein, all technical and scientific terms used herein have the same meaning as commonly understood by those of ordinary skill in the art.

Unless otherwise stated, the terms used in the specification and claims have the following meanings.

The three-letter and single-letter codes for amino acids used in the present disclosure are as described in *J. Biol. Chem.,* 243, p3558 (1968).

The term "amino acid" refers to naturally occurring and synthetic amino acids, as well as amino acid analogs and amino acid mimics that function similarly to the naturally occurring amino acids. Naturally occurring amino acids are those encoded by genetic codes and those amino acids later modified, e.g., hydroxyproline, γ-carboxyglutamic acid, and O-phosphoserine. Amino acid analogs refer to compounds that have a substantially identical chemical structure (i.e., an α carbon that binds to hydrogen, carboxyl, amino, and an R group) to naturally occurring amino acids, e.g., homoserine, norleucine, methionine sulfoxide, and methioninemethyl sulfonium. Such analogs have a modified R group (e.g., norleucine) or a modified peptide skeleton, but retain an identical basic chemical structure to naturally occurring amino acids. Amino acid mimics refer to chemical compounds that have a structure different from the general chemical structure of amino acids, but function similarly to naturally occurring amino acids.

The term "amino acid mutation" includes amino acid substitutions (also known as amino acid replacements), deletions, insertions, and modifications. Any combination of substitutions, deletions, insertions, and modifications can be made to obtain a final construct, as long as the final construct possesses the desired properties, such as reduced binding to the Fc receptor. Amino acid sequence deletions and insertions include deletions and insertions at the amino terminus and/or the carboxyl terminus of a polypeptide chain. Specific amino acid mutations may be amino acid substitutions. In one embodiment, the amino acid mutation is a non-conservative amino acid substitution, i.e., the replacement of one amino acid with another amino acid having different structural and/or chemical properties. Amino acid substitutions include replacement with non-naturally occurring amino acids or with derivatives of the 20 natural amino acids (e.g., 4-hydroxyproline, 3-methylhistidine, ornithine, homoserine, and 5-hydroxylysine). Amino acid mutations can be generated using genetic or chemical methods well known in the art. Genetic methods may include site-directed mutagenesis, PCR, gene synthesis, and the like. It is contemplated that methods for altering amino acid side chain groups other than genetic engineering, such as chemical modification, may also be used. Various names may be used herein to indicate the same amino acid mutation. Herein, the expression of "position + amino acid residue" may be used to denote an amino acid residue at a specific position. For example, 366W indicates that an amino acid residue at position 366 is W. T366W indicates that the amino acid residue at position 366 is substituted from the original T to W.

The term "antibody" is used in the broadest sense and encompasses a variety of antibody structures, including but not limited to monoclonal antibodies, polyclonal antibodies, monospecific antibodies, multispecific antibodies (e.g., bispecific antibodies), full-length antibodies, and antibody fragments (or antigen-binding fragments, or antigen-binding moieties), so long as they exhibit the desired antigen-binding activity. An intact antibody generally comprises two light

chains and two heavy chains. From the N-terminus to the C-terminus, each heavy chain comprises one variable region (VH), also known as variable heavy domain or heavy chain variable region, followed by three constant domains (CH1, CH2, and CH3). Similarly, from the N-terminus to the C-terminus, each light chain comprises one variable region (VL), also known as variable light domain or light chain variable domain, followed by one constant light domain (light chain constant region, CL). The terms "full-length antibody", "intact antibody", and "whole antibody" are used interchangeably herein and refer to an antibody having a substantially similar structure to a native antibody structure or whose heavy chains have an Fc region. In a native intact antibody, the light chain comprises a light chain variable region VL and a constant region CL, wherein the VL is positioned at the amino terminus of the light chain, and the light chain constant region comprises a κ chain and a λ chain; the heavy chain comprises a variable region VH and constant regions (CH1, CH2, and CH3), wherein the VH is positioned at the amino terminus of the heavy chain, and the constant regions are positioned at the carboxyl terminus, wherein the CH3 is closest to the carboxyl terminus, and the heavy chain can be of any isotype, including IgG (including subtypes IgG1, IgG2, IgG3, and IgG4), IgA (including subtypes IgA1 and IgA2), IgM, and IgE.

The term antibody "variable region" or "variable domain" refers to a domain in the heavy or light chain of an antibody that is involved in the binding of the antibody to an antigen. Herein, the heavy chain variable region (VH) and light chain variable region (VL) of the antibody each comprise four conserved framework regions (FRs) and three complementarity determining regions (CDRs). The term "complementarity determining region" or "CDR" refers to a region in the variable domain that primarily contributes to antigen binding; "framework" or "FR" refers to variable domain residues other than CDR residues. A VH comprises 3 CDRs: HCDR1, HCDR2, and HCDR3; a VL comprises 3 CDRs: LCDR1, LCDR2, and LCDR3. Each VH and VL is composed of three CDRs and four FRs arranged from the amino terminus (also known as N-terminus) to the carboxyl terminus (also known as C-terminus) in the following order: FR1, CDR1, FR2, CDR2, FR3, CDR3, and FR4.

The amino acid sequence boundaries of the CDRs can be determined by a variety of well-known schemes, for example, the "Kabat" numbering scheme (see Kabat et al., (1991), Sequences of Proteins of Immunological Interest, 5th Edition, Public Health Service, National Institutes of Health, Bethesda, MD), the "Chothia" numbering scheme, the "ABM" numbering scheme, the "contact" numbering scheme (see Martin, ACR. Protein Sequence and Structure Analysis of Antibody Variable Domains [J]. 2001), and the ImMunoGenTics (IMGT) numbering scheme (Lefranc, M.P. et al., Dev. Comp. Immunol., 27, 55-77 (2003); Front Immunol. 2018 Oct 16; 9:2278), and the like. The corresponding relationships between the various numbering schemes are well known to those skilled in the art and, illustratively, are shown in Table 1 below.

Table 1. The relationships between CDR numbering schemes

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

Unless otherwise stated, the Kabat numbering scheme is applied to the sequences of the variable regions and CDRs in the present disclosure.

The term "antibody fragment" refers to a molecule different from an intact antibody, which comprises a moiety of an intact antibody that binds to an antigen to which the intact antibody binds. Examples of antibody fragments include, but are not limited to, Fv, Fab, Fab', Fab'-SH, $F(ab')_2$, single-domain antibodies, single-chain Fab (scFab), diabodies, linear antibodies, single-chain antibody molecules (e.g., scFv), and multispecific antibodies formed from antibody fragments.

The term "Fc region" or "fragment crystallizable region" is used to define the C-terminal region of the heavy chain of an antibody, including native Fc regions and engineered Fc regions. In some embodiments, the Fc region comprises two identical or different subunits. In some embodiments, the Fc region of the human IgG heavy chain is defined as extending from the amino acid residue at position Cys226 or from Pro230 to its carboxyl terminus. Suitable Fc regions used for the antibodies described herein include Fc regions of human IgG1, IgG2 (IgG2A and IgG2B), IgG3, and IgG4. In some embodiments, the boundaries of the Fc region may also be varied, for example, by deleting the C-terminal lysine of the Fc region (residue 447 according to the EU numbering scheme) or deleting the C-terminal glycine and lysine of the Fc region (residues 446 and 447 according to the EU numbering scheme). Unless otherwise stated, the numbering scheme for the Fc region is the EU numbering scheme, also known as the EU index.

The term "chimeric antibody" refers to an antibody in which a portion of the heavy and/or light chain in the antibody is derived from a particular source or species, while the remainder of the heavy and/or light chain is derived from another different source or species.

The term "humanized antibody" is an antibody that retains the reactivity of a non-human antibody while having low immunogenicity in humans. For example, the humanization can be achieved by retaining the non-human CDRs and replacing the remainder of the antibody with its human counterparts (i.e., the constant regions and the framework region portion of the variable regions).

The terms "human antibody", "human-derived antibody", "fully human antibody", and "complete human antibody" are used interchangeably and refer to antibodies in which the variable regions and constant regions are human sequences. The term encompasses antibodies that are derived from human genes but have, for example, sequences that reduce possible immunogenicity, increase affinity, eliminate cysteines or glycosylation sites that may cause undesired folding, or the like. The term encompasses antibodies recombinantly produced in non-human cells (that may confer glycosylation not characteristic of human cells). The term also encompasses antibodies that have been cultured in transgenic mice comprising human immunoglobulin heavy and light chain loci. The meaning of the human antibody specifically excludes humanized antibodies comprising non-human antigen-binding residues.

The term "affinity" refers to the overall strength of the non-covalent interaction between a single binding site of a molecule (e.g., an antibody) and its binding ligand (e.g., an antigen). Unless otherwise indicated, as used herein, binding "affinity" refers to an internal binding affinity that reflects the interaction between members of a binding pair (e.g., an antibody and an antigen). The affinity of molecule X for its ligand Y can be generally denoted by the dissociation constant (KD). Affinity can be determined by conventional methods known in the art, including those described herein.

As used herein, the term "kassoc" or "ka" refers to the association rate of a particular antibody-antigen interaction, while the term "kdis" or "kd" refers to the dissociation rate of a particular antibody-antigen interaction. The term "KD" refers to the dissociation constant, which is obtained from the ratio of kd to ka (i.e., kd/ka) and denoted by a molar concentration (M). The KD value of an antibody can be determined using methods well known in the art. For example, surface plasmon resonance is determined using a biosensing system such as a system, or affinity in a solution is determined by solution equilibrium titration (SET).

The term "effector function" refers to biological activities that can be attributed to the Fc region of an antibody (either the natural sequence Fc region or the amino acid sequence variant Fc region) and vary with the antibody isotype. Examples of effector functions of an antibody include, but are not limited to: C1q binding and complement-dependent cytotoxicity, Fc receptor binding, antibody-dependent cell-mediated cytotoxicity (ADCC), phagocytosis, down-regulation of cell surface receptors (e.g., B cell receptors), and B cell activation.

The term "monoclonal antibody" refers to a population of substantially homogeneous antibodies, that is, the amino acid sequences of the antibody molecules comprised in the population are identical, except for a small number of natural mutations that may exist. In contrast, a polyclonal antibody formulation generally comprises several different antibodies comprising different amino acid sequences in their variable domains, which are generally specific for different epitopes. "Monoclonal" should not be construed as requiring the production of the antibody by any particular method. In some embodiments, the antibody provided by the present disclosure is a monoclonal antibody.

The term "antigen" refers to a molecule or a portion of a molecule that is capable of being bound by a selective binding agent, such as an antigen-binding protein (including, for example, an antibody). An antigen may have one or more epitopes capable of interacting with different antigen-binding proteins (e.g., antibodies).

The term "epitope" refers to an area or region on an antigen that is capable of specifically binding to an antibody or an antigen-binding fragment thereof. Epitopes can be formed from contiguous strings of amino acids (linear epitope) or comprise non-contiguous amino acids (conformational epitope), e.g., coming in spatial proximity due to the folding of the antigen (i.e., by the tertiary folding). The difference between the conformational epitope and the linear epitope is that in the presence of denaturing solvents, the binding of the antibody to the conformational epitope is undetectable. An epitope comprises at least 3, at least 4, at least 5, at least 6, at least 7, or 8-10 amino acids in a unique spatial conformation. Screening for antibodies that bind to particular epitopes (i.e., those that bind to identical epitopes) can be performed using routine methods in the art, including, for example, but not limited to, alanine scanning, peptide blotting, peptide cleavage analysis, epitope excision, epitope extraction, chemical modification of the antigen (Prot. Sci. 9 (2000) 487-496), and cross-blocking.

The term "capable of specifically binding", "specifically binding", or "binding" means that an antibody is capable of binding to a certain antigen or an epitope thereof with higher affinity than to other antigens or epitopes. Generally, an antibody binds to an antigen or an epitope thereof with an equilibrium dissociation constant (KD) of about $1 \times 10^{-7}$ M or less (e.g., about $1 \times 10^{-8}$ M or less). In some embodiments, the KD for the binding of an antibody to an antigen is 10% or less (e.g., 1%) of the KD for the binding of the antibody to a non-specific antigen (e.g., BSA or casein). KD may be determined using known methods, for example, by a BIACORE® surface plasmon resonance assay. However, an antibody binding specifically to an antigen or an epitope thereof may have cross-reactivity to other related antigens, e.g., to corresponding antigens from other species (homologous), such as humans or monkeys, e.g., Macaca fascicularis (cynomolgus, cyno),

Pan troglodytes (chimpanzee, chimp), or Callithrix jacchus (commonmarmoset, marmoset). The term "nucleic acid" is used interchangeably herein with the term "polynucleotide" and refers to deoxyribonucleotide or ribonucleotide and a polymer thereof in either single-stranded or double-stranded form. The term encompasses nucleic acids comprising known nucleotide analogs or modified backbone residues or linkages. The nucleic acids are synthetic, naturally occurring, and non-naturally occurring, have similar binding properties to the reference nucleic acid, and are metabolized in a manner similar to the reference nucleotide. Examples of such analogs include, but are not limited to, phosphorothioate, phosphoramidate, methylphosphonate, chiral-methylphosphonate, 2-O-methyl ribonucleotide, and peptide-nucleic acid (PNA). "Isolated" nucleic acid refers to a nucleic acid molecule that has been separated from components of its natural environment. The isolated nucleic acid includes a nucleic acid molecule comprised in a cell that generally comprises the nucleic acid molecule, but the nucleic acid molecule is present extrachromosomally or at a position different from its natural chromosomal position. An isolated nucleic acid encoding a polypeptide or a fusion protein refers to one or more nucleic acid molecules encoding the polypeptide or fusion protein, including such one or more nucleic acid molecules in a single vector or separate vectors, and such one or more nucleic acid molecules present at one or more positions in a host cell. Unless otherwise stated, a particular nucleic acid sequence also implicitly encompasses conservatively modified variants thereof (e.g., degenerate codon substitutions) and complementary sequences, as well as the sequence explicitly indicated. Specifically, as detailed below, degenerate codon substitutions may be obtained by generating sequences in which the third position of one or more selected (or all) codons is substituted with mixed bases and/or deoxyinosine residues.

The terms "polypeptide" and "protein" are used interchangeably herein and refer to a polymer of amino acid residues in which one or more amino acid residues are artificial chemical mimics of corresponding naturally occurring amino acids, as well as to naturally occurring amino acid polymers and non-naturally occurring amino acid polymers. Unless otherwise stated, a particular polypeptide sequence also implicitly encompasses conservatively modified variants thereof.

The term sequence "identity" refers to the degree (percentage) to which the amino acids/nucleic acids of two sequences are identical at equivalent positions; when the two sequences are optimally aligned, gaps are introduced as necessary to achieve the maximum percent identity, and any conservative substitution is not seen as part of the sequence identity. To determine percent sequence identity, alignments can be accomplished by techniques known to those skilled in the art, for example, using publicly available computer software, such as BLAST, BLAST-2, ALIGN, ALIGN-2, or Megalign (DNASTAR) software. Those skilled in the art can determine parameters suitable for measuring alignment, including any algorithms required to achieve maximum alignment of the full length of the aligned sequences.

The term "vector" means a polynucleotide molecule capable of transporting another polynucleotide linked thereto. One type of vector is a "plasmid", which refers to a circular double-stranded DNA loop into which additional DNA segments can be ligated. Another type of vector is a viral vector, such as an adeno-associated viral vector (AAV or AAV2), wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. The term "expression vector" or "expression construct" refers to a vector that can transform a host cell and comprises a nucleic acid sequence that directs and/or controls the expression of one or more heterologous coding regions operably linked thereto. Expression constructs may include, but are not limited to, sequences that affect or control transcription and translation and affect RNA splicing of a coding region operably linked thereto in the presence of an intron.

The terms "host cell", "host cell line", and "host cell culture" are used interchangeably and refer to cells into which exogenous nucleic acids have been introduced, including progenies of such cells. Host cells include "transformants" and "transformed cells", which include primary transformed cells and progenies derived therefrom, regardless of the number of passages. Progeny may not be exactly the same as parent cells in terms of nucleic acid content and may contain mutations. Mutant progenies that have the same function or biological activity as the cells screened or selected from the initially transformed cells are included herein. Host cells include prokaryotic and eukaryotic host cells, wherein the eukaryotic host cells include, but are not limited to, mammalian cells, insect cell lines, plant cells, and fungal cells. Exemplary host cells are as follows: Chinese hamster ovary (CHO) cells, NSO, SP2 cells, HeLa cells, baby hamster kidney (BHK) cells, monkey kidney cells (COS), human hepatocellular carcinoma cells (e.g., HepG2), A549 cells, 3T3 cells and HEK-293 cells, Pichiapastoris, Pichia finlandica, Candida albicans, Aspergillus niger, Aspergillus oryzae, and Trichoderma reesei.

The term "alkyl" refers to a saturated straight-chain or branched-chain aliphatic hydrocarbon group having 1 to 20 (e.g., 1, 2, 3, 4, 5, 6, 7, 8, 9, 10, 11, 12, 13, 14, 15, 16, 17, 18, 19, or 20) carbon atoms (i.e., $C_{1-20}$ alkyl). Preferably, the alkyl is an alkyl group having 1 to 12 carbon atoms (i.e., $C_{1-12}$ alkyl); more preferably, the alkyl is an alkyl group having 1 to 6 carbon atoms (i.e., $C_{1-6}$ alkyl). Non-limiting examples include: methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, various branched-chain isomers thereof, and the like. Alkyl may be substituted or unsubstituted, and when it is substituted, it may be substituted at any accessible point of attachment, and the substituent is preferably selected from the group consisting of one or more of a deuterium (D) atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkylene" refers to a divalent alkyl group, wherein the alkyl is as defined above; the alkylene has 1 to 20 (e.g., 1, 2, 3, 4, 5, 6, 7, 8, 9, 10, 11, 12, 13, 14, 15, 16, 17, 18, 19, or 20) carbon atoms (i.e., $C_{1-20}$ alkylene). Preferably, the alkylene is an alkylene group having 1 to 12 carbon atoms (i.e., $C_{1-12}$ alkylene); more preferably, the alkylene is an alkylene group having 1 to 6 carbon atoms (i.e., $C_{1-6}$ alkylene). Non-limiting examples include: $-CH_2-$, $-CH(CH_3)-$, $-C(CH_3)_2-$, $-CH_2CH_2-$, $-CH(CH_2CH_3)-$, $-CH_2CH(CH_3)-$, $-CH_2C(CH_3)_2-$, $-CH_2CH_2CH_2-$, $-CH_2CH_2CH_2CH_2-$, and the like. Alkylene may be substituted or unsubstituted, and when it is substituted, it may be substituted at any accessible point of attachment, and the substituent is preferably selected from the group consisting of one or more of a deuterium atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkoxy" refers to -O-(alkyl), wherein the alkyl is as defined above. Non-limiting examples include: methoxy, ethoxy, propoxy, butoxy, and the like. Alkoxy may be substituted or unsubstituted, and when it is substituted, it may be substituted at any accessible point of attachment, and the substituent is preferably selected from the group consisting of one or more of a deuterium atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "cycloalkyl" refers to a saturated or partially unsaturated monocyclic all-carbon ring (i.e., monocyclic cycloalkyl) or polycyclic system (i.e., polycyclic cycloalkyl) having 3 to 20 (e.g., 3, 4, 5, 6, 7, 8, 9, 10, 11, 12, 13, 14, 15, 16, 17, 18, 19, or 20) ring atoms (i.e., 3- to 20-membered cycloalkyl). Preferably, the cycloalkyl is a cycloalkyl group having 3 to 12 ring atoms (i.e.,3- to 12-membered cycloalkyl); more preferably, the cycloalkyl is a cycloalkyl group having 3 to 8 ring atoms (i.e., 3- to 8-membered cycloalkyl); most preferably, the cycloalkyl is a cycloalkyl group having 3 to 6 ring atoms (i.e., 3- to 6-membered cycloalkyl).

Non-limiting examples of the monocyclic cycloalkyl include: cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like.

The polycyclic cycloalkyl includes: spirocycloalkyl, fused cycloalkyl, and bridged cycloalkyl.

The compounds of the present disclosure may exist in specific stereoisomeric forms. The term "stereoisomer" refers to isomers that are structurally identical but differ in the arrangement of the atoms in space. It includes cis and trans (or *Z* and *E*) isomers, (-)- and (+)-isomers, (*R*)- and (*S*)-enantiomers, diastereomers, (*D*)- and (*L*)-isomers, tautomers, atropisomers, conformers, and mixtures thereof (e.g., mixtures of racemates and diastereomers). Additional asymmetric atoms may be present in the substituents in the compounds of the present disclosure. All such stereoisomers and mixtures thereof are included within the scope of the present disclosure. Optically active (-)- and (+)-isomers, (*R*)- and (*S*)-enantiomers, and (*D*)- and (*L*)-isomers can be prepared by chiral synthesis, chiral reagents, or other conventional techniques. One isomer of a certain compound of the present disclosure may be prepared by asymmetric synthesis or with a chiral auxiliary, or, when the molecule contains a basic functional group (e.g., amino) or an acidic functional group (e.g., carboxyl), a diastereomeric salt is formed with an appropriate optically active acid or base, followed by diastereomeric resolution by conventional methods known in the art to give the pure isomer. In addition, the separation of enantiomers and diastereomers is generally accomplished by chromatography.

In the chemical structure of the compound of the present disclosure, the bond "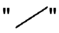" indicates an unspecified configuration; that is, if chiral isomers exist in the chemical structure, the bond "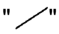" may be "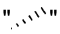" or "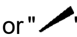", or includes both the configurations "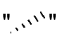" and "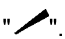".

The compounds of the present disclosure include all suitable isotopic derivatives of the compounds thereof. The term "isotopic derivative" refers to a compound in which at least one atom is replaced with an atom having the same atomic number but a different atomic mass. Examples of isotopes that can be incorporated into the compounds of the present disclosure include stable and radioactive isotopes of hydrogen, carbon, nitrogen, oxygen, phosphorus, sulfur, fluorine, chlorine, bromine, iodine, etc., such as $^{2}H$ (deuterium, D), $^{3}H$ (tritium, T), $^{11}C$, $^{13}C$, $^{14}C$, $^{15}N$, $^{17}O$, $^{18}O$, $^{32}p$, $^{33}p$, $^{33}S$, $^{34}S$, $^{35}S$, $^{36}S$, $^{18}F$, $^{36}Cl$, $^{82}Br$, $^{123}I$, $^{124}I$, $^{125}I$, $^{129}I$, and $^{131}I$, deuterium is preferred.

Compared to non-deuterated drugs, deuterated drugs have the advantages of reduced toxic and side effects, increased drug stability, enhanced efficacy, prolonged biological half-lives, and the like. All isotopic variations of the compound of the present disclosure, whether radioactive or not, are included within the scope of the present disclosure.

Each available hydrogen atom attached to a carbon atom may be independently replaced with a deuterium atom, wherein the replacement with deuterium may be partial or complete. The partial replacement with deuterium refers to the replacement of at least one hydrogen atom with at least one deuterium atom.

The term "antibody-drug conjugate (ADC)" refers to an antibody or an antigen-binding fragment thereof conjugated, via a linker unit, to a biologically active cytotoxin or a small-molecule drug with cell killing activity.

The antibody or the antibody fragment described herein may be conjugated to an effector molecule by any means. For example, the antibody or the antibody fragment may be chemically or recombinantly attached to the cytotoxic drug. Chemical means for preparing fusions or conjugates are known in the art and can be used to prepare immunoconjugates. The method for conjugating the antibody or the antibody fragment and the drug must be capable of linking the antibody to the cytotoxic drug without interfering with the ability of the antibody or the antibody fragment to bind to the target molecule.

In one embodiment, both the antibody and cytotoxic drug are proteins and can be conjugated using techniques well known in the art. There are hundreds of cross-linking agents disclosed in the art that can conjugate two proteins. The cross-linking agent is generally selected based on reactive functional groups available or inserted on the antibody or cytotoxic drug. Alternatively, if no reactive groups are present, a photo-activatable cross-linking agent may be used. In some cases, it may be desirable to include a spacer between the antibody and the cytotoxic drug. Cross-linking agents known in the art include homobifunctional agents: glutaraldehyde, dimethyl adipimidate, and bis(diazobenzidine), and heterobifunctional agents: m-maleimidobenzoyl-N-hydroxysuccinimide and sulfo-m-maleimidobenzoyl-N-hydroxysuccinimide.

Cross-linking agents that can be used to conjugate an effector molecule to an antibody fragment include, for example, TPCH (S-(2-thiopyridyl)-L-cysteine hydrazide) and TPMPH (S-(2-thiopyridyl)mercapto-propionohydrazide). TPCH and TPMPH react on the carbohydrate moiety of the glycoprotein that had previously been oxidized by mild periodate treatment, thereby forming a hydrazone bond between the hydrazide moiety of the cross-linking agent and the aldehyde generated by periodate. The heterobifunctional cross-linking agents GMBS (N-(γ-maleimidobutyryloxy)-succinimide) and SMCC (succinimidyl 4-(N-maleimido-methyl)cyclohexane) are reacted with a primary amine, thereby introducing a maleimido group onto the component. This maleimido group may then react with a sulfhydryl group on another component which may be introduced by a cross-linking agent, thereby forming a stable thioether bond between the components. If steric hindrance between the components interferes with the activity of either component, a cross-linking agent may be used to introduce a long spacer between the components, such as N-succinimidyl 3-(2-pyridyldithio) propionate (SPDP). Thus, there are many suitable cross-linking agents that may be used and selected individually depending on their effect on the yield of the optimal immunoconjugate.

The term "drug loading", also referred to as drug-to-antibody ratio (DAR), refers to the average number of drugs conjugated to each antibody in an ADC. It may range, for example, from about 1 to about 10 drugs conjugated to each antibody, and in certain embodiments, from about 1 to about 8 drugs conjugated to each antibody, preferably selected from the group consisting of 2-8, 2-7, 2-6, 2-5, 2-4, 3-4, 3-5, 4-8, 5-6, 5-7, 5-8, and 6-8 drugs conjugated to each antibody. Illustratively, the drug loading may be an average of 1, 2, 3, 4, 5, 6, 7, 8, 9, or 10. The ADC general formulas of the present disclosure include a set of antibody-drug conjugates within a certain range as described above. In the embodiments of the present disclosure, drug loading may be represented as n and is a decimal or an integer. Drug loading can be determined by conventional methods such as UV/visible spectroscopy, mass spectrometry, ELISA assays, HIC, and RP-HPLC. The term "linker unit" or "linker fragment" refers to a chemical structure fragment or bond that is linked to an antibody or an antigen-binding fragment thereof at one end and to a drug at the other end, and it may also be linked to a drug after being linked to another linker.

The term "linker" may comprise one or more linker components. Exemplary linker components include 6-maleimidocaproyl ("MC"), maleimidopropionyl ("MP"), valine-citrulline ("val-cit" or "vc"), alanine-phenylalanine ("ala-phe"), p-aminobenzyloxycarbonyl ("PAB"), and those derived from coupling to a linker reagent: N-succinimidyl 4-(2-pyridylthio)valerate ("SPP"), N-succinimidyl 4-(N-maleimidomethyl)cyclohexane-1 carboxylate ("SMCC", also referred to herein as "MCC"), and N-succinimidyl(4-iodo-acetyl)aminobenzoate ("SIAB"). The linker may include stretcher units, spacer units, and amino acid units, and may be synthesized using methods known in the art, such as those described in US2005-0238649A1. The linker may be a "cleavable linker" favorable for the release of drugs in cells. For example, acid-labile linkers (e.g., hydrazones), protease-sensitive (e.g., peptidase-sensitive) linkers, photolabile linkers, dimethyl linkers, or disulfide-containing linkers may be used (Chari et al., Cancer Research, 52: 127-131(1992); U.S. Patent No. 5,208,020).

Linker components include, but are not limited to:

MC = 6-maleimidocaproyl, whose structure is shown below:

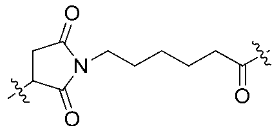

,

Val-Cit or "vc" = valine-citrulline (an exemplary dipeptide in a protease cleavable linker), citrulline = 2-amino-5-ureidopentanoic acid,

PAB group = p-aminobenzyloxycarbonyl (an example of "self-immolative" linker components), whose structure is shown below:

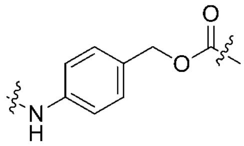

,

Me-Val-Cit = N-methyl-valine-citrulline (where the linker peptide bond has been modified to prevent it from being cleaved by cathepsin B),

MC(PEG)6-OH = maleimidocaproyl-polyethylene glycol (attachable to antibody cysteine),

SPP = N-succinimidyl 4-(2-pyridylthio)valerate,

SPDP = N-succinimidyl 3-(2-pyridyldithio)propionate,

SMCC = succinimidyl-4-(N-maleimidomethyl)cyclohexane-1-carboxylate, and

IT = iminothiolane.

The linker unit-compound of the present disclosure reacts with an antibody having a sulfhydryl group to give the antibody-drug conjugate of the present disclosure).

The antibody having a sulfhydryl group can be obtained using methods well known to those skilled in the art (Hermanson, G. T, Bioconjugate Techniques, pp. 56-136, pp. 456-493, Academic Press (1996)). For example, the following methods can be used: reacting the amino group of an antibody with a Traut's reagent; reacting the amino group of an antibody with N-succinimidyl S-acetylthioalkanoate, and then with hydroxylamine; reacting an antibody with N-succinimidyl 3-(pyridyldithio)propionate, and then reacting with a reductant; reacting an antibody with a reductant, such as dithiothreitol, 2-mercaptoethanol, tris(2-carboxyethyl)phosphine hydrochloride (TCEP), and the like, to reduce a disulfide bond in a hinge part in the antibody to produce a sulfhydryl group; and the like. The methods are not limited to these methods.

In one embodiment of the present disclosure, the cytotoxic drug is conjugated to a sulfhydryl group of the antibody via a linker unit.

The loading of the antibody-drug conjugate can be controlled by the following non-limiting methods, including:

(1) controlling a molar ratio of a linking moiety of the compound to the monoclonal antibody,
(2) controlling reaction time and temperature, and
(3) selecting different reaction reagents.

The term "optionally" or "optional" means that the event or circumstance subsequently described may, but does not necessarily, occur, and this description includes instances where the event or circumstance occurs or does not occur. For example, "$C_{1-6}$ alkyl optionally substituted with halogen or cyano" means that the halogen or cyano may, but does not necessarily, exist, and this description includes an instance where the alkyl is substituted with halogen or cyano and an instance where the alkyl is not substituted with halogen or cyano.

The term "substituted" means that one or more, preferably 1 to 6, and more preferably 1 to 3 hydrogen atoms in the group are independently substituted with a corresponding number of substituents. Those skilled in the art can determine (experimentally or theoretically) possible or impossible substitutions without undue effort. For example, the binding of an amino or hydroxy group having free hydrogen to a carbon atom having an unsaturated (e.g., olefinic) bond may be unstable.

The term "pharmaceutically acceptable salt" refers to a salt of the compound of the present disclosure, which may be selected from the group consisting of inorganic and organic salts. Such salts are safe and effective when used in the body of a mammal and possess the requisite biological activity. The salts may be prepared separately during the final separation and purification of the compound, or by reacting an appropriate group with an appropriate base or acid. Bases commonly used to form pharmaceutically acceptable salts include inorganic bases such as sodium hydroxide and

potassium hydroxide, and organic bases such as ammonia. Acids commonly used to form pharmaceutically acceptable salts include inorganic acids and organic acids.

In the present disclosure, the concentration of the antibody-drug conjugate is expressed in terms of the concentration of the protein, i.e., the concentration of the antibody moiety in the antibody-drug conjugate.

All compounds in the present disclosure can be drawn as form A or form B. All tautomeric forms are within the scope of the present disclosure. The names of the compounds do not exclude any tautomers.

"Prodrug" refers to a substance that can be converted *in vivo* under physiological conditions, e.g., by hydrolysis in blood, to generate the active parent drug compound.

The term "pharmaceutically acceptable" used herein means that those compounds, materials, compositions, and/or dosage forms are, within the scope of reasonable medical judgment, suitable for use in contact with the tissues of subjects without excessive toxicity, irritation, allergic reaction, or other problems or complications, and are commensurate with a reasonable benefit/risk ratio and effective for the intended use.

For the manufacture of conventional pharmaceutical compositions, reference can be made to pharmacopeias (such as the Chinese Pharmacopoeia) or specifications approved by drug regulatory authorities.

As used herein, the singular forms "a", "an", and "the" include plural references and vice versa, unless otherwise clearly defined in the context.

Unless otherwise clearly stated in the context, throughout the description and the claims, the words "comprise", "have", "include", and the like, should be construed in an inclusive sense as opposed to an exclusive or exhaustive sense.

The term "and/or" is meant to include the two meanings "and" and "or". For example, the phrase "A, B, and/or C" is intended to encompass each of the following: A, B, and C; A, B, or C; A or C; A or B; B or C; A and C; A and B; B and C; A (alone); B (alone); and C (alone). When a trade name is used in the present disclosure, it is intended to include the formulation of the commercial product under the trade name, and the drug and active drug component of the commercial product under the trade name.

"About" means that it is within an acceptable error range for a particular value as determined by one of ordinary skill in the art, which will depend in part on how the value is measured or determined, i.e., the limitations of the measurement system. In the context of a particular assay, result, or embodiment, "about" means that it is within one standard deviation according to the practice in the art, unless otherwise explicitly stated in the example or elsewhere in the specification.

The term "pharmaceutically acceptable carrier" refers to an ingredient in a pharmaceutical formulation that is different from the active ingredient and is not toxic to the subject. Pharmaceutically acceptable carriers include, but are not limited to, buffers, excipients, stabilizers, or preservatives.

The term "package insert" is used to refer to instructions generally included in commercial packages of therapeutic products, which contain information about the indications, usage, dosage, administration, combination therapy, contraindications, and/or warnings concerning the use of such therapeutic products.

The term "subject" or "individual" includes humans and non-human animals. Non-human animals include all vertebrates (e.g., mammals and non-mammals) such as non-human primates (e.g., cynomolgus monkeys), sheep, dogs, cows, chickens, amphibians, and reptiles. Unless indicated, the terms "patient" and "subject" are used interchangeably herein. As used herein, the term "cynomolgus monkey (cyno)" or "cynomolgus" refers to *Macaca fascicularis.* In certain embodiments, the individual or subject is a human.

The term "excipient" is an addition, besides the main drug, to a pharmaceutical formulation. It may also be referred to as an auxiliary material. For example, binders, fillers, disintegrants, and lubricants in tablets, the matrix part in semi-solid ointment and cream preparations, preservatives, antioxidants, corrigents, fragrances, cosolvents, emulsifiers, solubilizers, osmotic pressure regulators, colorants, and the like in liquid formulations can all be referred to as excipients.

The term "diluent", also referred to as a filler, is used primarily to increase the weight and volume of the tablet. The addition of a diluent not only ensures a certain volume, but also reduces the dose deviation of the main ingredients and improves the compression moldability, etc., of the drug. When a drug in tablet form contains oily components, an absorbent is necessarily added to absorb the oily components so as to maintain a "dry" state that facilitates the preparation of tablets. Examples include starch, lactose, inorganic salts of calcium, microcrystalline cellulose, and the like.

The term "pharmaceutical composition" refers to a mixture containing one or more of the compounds or the physiologically/pharmaceutically acceptable salts or pro-drugs thereof described herein, and other chemical components, for example, physiologically/pharmaceutically acceptable carriers and excipients. The pharmaceutical composition is intended to promote administration to an organism and facilitate the absorption of the active ingredient so that it can exert its biological activity.

The pharmaceutical composition may be in the form of a sterile injectable aqueous solution. Available and acceptable vehicles and solvents include water, Ringer's solution, and isotonic sodium chloride solution. The sterile injectable formulation may be a sterile injectable oil-in-water microemulsion in which the active ingredient is dissolved in the oil phase. For example, the active ingredient is dissolved in a mixture of soybean oil and lecithin. The oil solution is then added to a mixture of water and glycerol and treated to form a microemulsion. The injection or microemulsion can be locally

injected into the bloodstream of a subject in large quantities. Alternatively, it may be desirable to administer the solution and microemulsion in such a way that maintains a constant circulating concentration of the compound of the present disclosure. To maintain such a constant concentration, a continuous intravenous delivery device may be used. An example of such a device is a Deltec CADD-PLUS. TM. 5400 intravenous injection pump. The pharmaceutical composition may be in the form of a sterile injectable aqueous or oily suspension for intramuscular and subcutaneous administration. The suspension can be prepared according to the prior art using those suitable dispersants or wetting agents and suspending agents as described above. The sterile injectable formulation may also be a sterile injection or suspension prepared in a parenterally acceptable non-toxic diluent or solvent, e.g., a solution prepared in 1,3-butanediol. In addition, a sterile fixed oil may be conventionally used as a solvent or a suspending medium. For this purpose, any blend fixed oil including synthetic monoglycerides or diglycerides can be used. In addition, fatty acids such as oleic acid may also be used in the preparation of injections.

"Administrating" or "giving", when applied to animals, humans, experimental subjects, cells, tissues, organs, or biological fluids, refers to contact of an exogenous drug, therapeutic agent, diagnostic agent, or composition with the animals, humans, subjects, cells, tissues, organs, or biological fluids.

The term "sample" refers to a collection of similar fluids, cells, or tissues isolated from a subject, as well as fluids, cells, or tissues present within a subject. Exemplary samples are biological fluids (such as blood; serum; serosal fluids; plasma; lymph; urine; saliva; cystic fluids; tears; excretions; sputum; mucosal secretions of secretory tissue and organs; vaginal secretions; ascites; fluids in the pleura, pericardium, peritoneum, abdominal cavity, and other body cavities; fluids collected from bronchial lavage; synovial fluids; liquid solutions in contact with a subject or biological source, e.g., cell and organ culture media (including cell or organ conditioned culture media); lavage fluids; and the like), tissue biopsy samples, fine needle punctures, surgically excised tissues, organ cultures, or cell cultures.

The term "pharmaceutically acceptable salt" refers to a salt of the antibody-drug conjugates of the present disclosure, or a salt of the compound described in the present disclosure. Such salts are safe and effective when used in the body of a mammal and possess the required biological activity. The antibody-drug conjugates of the present disclosure contain at least one amino group and therefore can form salts with acids.

"Treatment" or "treat" (and grammatical variations thereof) refer to clinical intervention in an attempt to alter the natural course of the treated individual, which may be performed either for prophylaxis or during the course of clinical pathology. Desirable effects of the treatment include, but are not limited to, preventing the occurrence or recurrence of a disease, palliating symptoms, palliating/reducing any direct or indirect pathological consequences of the disease, preventing metastasis, decreasing the rate of disease progression, ameliorating or palliating the disease state, and regressing or improving prognosis. In some embodiments, an antibody of the present disclosure is used to delay the development of or slow the progression of a disease.

"Effective amount" is generally an amount sufficient to reduce the severity and/or frequency of symptoms, eliminate symptoms and/or underlying causes, prevent the appearance of symptoms and/or their underlying causes, and/or ameliorate or improve damage caused by or associated with a disease state. In some examples, the effective amount is a therapeutically effective amount or a prophylactically effective amount. "Therapeutically effective amount" is an amount sufficient to treat a disease state or symptom, particularly a state or symptom associated with the disease state, or to otherwise prevent, hinder, delay, or reverse the progression of the disease state or any other undesirable symptoms associated with the disease in any way. "Prophylactically effective amount" is an amount that, when administered to a subject, will have a predetermined prophylactic effect, e.g., preventing or delaying the onset (or recurrence) of the disease state, or reducing the likelihood of the onset (or recurrence) of the disease state or associated symptoms. Complete treatment or prevention does not necessarily occur after administration of one dose and may occur after administration of a series of doses. Thus, a therapeutically or prophylactically effective amount may be administered in one or more doses. "Therapeutically effective amount" and "prophylactically effective amount" may vary depending on the following factors: e.g., the disease state, age, sex, and weight of the individual, and the ability of a therapeutic agent or combination of therapeutic agents to elicit a desired response in the individual. Exemplary indicators of an effective therapeutic agent or combination of therapeutic agents include, for example, improved health condition of a subject.

One or more embodiments of the present disclosure are described in detail in the specification above. Other features, objects, and advantages of the present disclosure will be apparent from the specification and the claims. In the specification and claims, singular forms include plural referents unless otherwise indicated clearly in the context. Unless otherwise defined, all technical and scientific terms used herein have the meanings generally understood by those of ordinary skill in the art to which the present disclosure belongs. All the patents and publications cited in the specification are incorporated by reference. The following examples are set forth in order to more fully illustrate the preferred embodiments of the present disclosure. These examples should not be construed in any way as limiting the scope of the present disclosure, which is defined by the claims. One or more embodiments of the present disclosure are described in detail in the specification above. Although any methods and materials similar or identical to those described herein can be used in the practice or testing of the present disclosure, the preferred methods and materials are described below. Other features, objects, and advantages of the present disclosure will be apparent from the specification and the claims. In the

specification and claims, singular forms include plural referents unless otherwise indicated clearly in the context. Unless otherwise defined, all technical and scientific terms used herein have the meanings generally understood by those of ordinary skill in the art to which the present disclosure belongs. All the patents and publications cited in the specification are incorporated by reference. The following examples are set forth in order to more fully illustrate the preferred embodiments of the present disclosure. These examples should not be construed in any way as limiting the scope of the present disclosure, which is defined by the claims.

Experimental methods without specific conditions indicated in the examples or test examples of the present disclosure were generally conducted under conventional conditions or conditions recommended by the manufacturer of the starting materials or commercial products. See Sambrook et al., Molecular Cloning: A Laboratory Manual, Cold Spring Harbor Laboratory Press; Current Protocols in Molecular Biology, Ausubel et al., Greene Publishing Association, Wiley Interscience, NY. Reagents without specific sources indicated were commercially available conventional reagents.

## I. Antibody Preparation

### Example 1: Construction of Cell Strains CHO-K1 Highly Expressing PSMA

pCDH-huPSMA or pCDH-cynoPSMA lentiviral expression vector plasmids, together with pVSV-G and pCMV-dR8.91 lentiviral system packaging vectors, were transfected into 293T cells using Lipofectamine 3000 transfection reagent. The medium supernatant containing viruses was collected, filtered, and centrifuged at ultra-high speed. After the supernatant was discarded, the residue was resuspended in 1 mL of sterile PBS. Chinese hamster ovary cells CHO-K1 were infected with the concentrated virus, screened using puromycin for two to three weeks, and subjected to FACS single-cell sorting.

According to the PSMA expression levels on the surface of CHO-K1 cells infected with lentivirus determined by FACS, monoclonal cell strains huPSMA-CHO-K1 and cynoPSMA-CHO-K1 with high PSMA expression levels were selected. The selected monoclonal cell strains were expanded and preserved by freezing for subsequent experiments. The related amino acid sequences are as follows:

Amino acid sequence of human PSMA (UniProtKB - Q04609-1):

MWNLLHETDSAVATARRPRWLCAGALVLAGGFFLLGFLFGWFIKSSNEATNITP
KHNMKAFLDELKAENIKKFLYNFTQIPHLAGTEQNFQLAKQIQSQWKEFGLDS
VELAHYDVLLSYPNKTHPNYISIINEDGNEIFNTSLFEPPPPGYENVSDIVPPFSAF
SPQGMPEGDLVYVNYARTEDFFKLERDMKINCSGKIVIARYGKVFRGNKVKNA
QLAGAKGVILYSDPADYFAPGVKSYPDGWNLPGGGVQRGNILNLNGAGDPLTP
GYPANEYAYRRGIAEAVGLPSIPVHPIGYYDAQKLLEKMGGSAPPDSSWRGSLK
VPYNVGPGFTGNFSTQKVKMHIHSTNEVTRIYNVIGTLRGAVEPDRYVILGGHR
DSWVFGGIDPQSGAAVVHEIVRSFGTLKKEGWRPRRTILFASWDAEEFGLLGST
EWAEENSRLLQERGVAYINADSSIEGNYTLRVDCTPLMYSLVHNLTKELKSPDE
GFEGKSLYESWTKKSPSPEFSGMPRISKLGSGNDFEVFFQRLGIASGRARYTKN
WETNKFSGYPLYHSVYETYELVEKFYDPMFKYHLTVAQVRGGMVFELANSIVL
PFDCRDYAVVLRKYADKIYSISMKHPQEMKTYSVSFDSLFSAVKNFTEIASKFSE
RLQDFDKSNPIVLRMMNDQLMFLERAFIDPLGLPDRPFYRHVIYAPSSHNKYAG
ESFPGIYDALFDIESKVDPSKAWGEVKRQIYVAAFTVQAAAETLSEVA

SEQ ID NO: 1

Amino acid sequence of monkey PSMA (UniProtKB - A0A2K5VNZ0-1):

MWNLLHETDSAVATARRPRWLCAGALVLAGGFFLLGFLFGWFIKSSSEATNITP
KHNMKAFLDELKAENIKKFLHNFTQIPHLAGTEQNFQLAKQIQSQWKEFGLDS
VELTHYDVLLSYPNKTHPNYISIINEDGNEIFNTSLFEPPPAGYENVSDIVPPFSAF
SPQGMPEGDLVYVNYARTEDFFKLERDMKINCSGKIVIARYGKVFRGNKVKNA
QLAGATGVILYSDPADYFAPGVKSYPDGWNLPGGGVQRGNILNLNGAGDPLTP
GYPANEYAYRRGIAEAVGLPSIPVHPIGYYDAQKLLEKMGGSASPDSSWRGSLK
VPYNVGPGFTGNFSTQKVKMHIHSTSEVTRIYNVIGTLRGAVEPDRYVILGGHR
DSWVFGGIDPQSGAAVVHEIVRSFGTLKKEGWRPRRTILFASWDAEEFGLLGST
EWAEENSRLLQERGVAYINADSSIEGNYTLRVDCTPLMYSLVYNLTKELESPDEG
FEGKSLYESWTKKSPSPEFSGMPRISKLGSGNDFEVFFQRLGIASGRARYTKNWE
TNKFSSYPLYHSVYETYELVEKFYDPMFKYHLTVAQVRGGMVFELANSVVLPF
DCRDYAVVLRKYADKIYNISMKHPQEMKTYSVSFDSLFSAVKNFTEIASKFSERL
RDFDKSNPILLRMMNDQLMFLERAFIDPLGLPDRPFYRHVIYAPSSHNKYAGESF
PGIYDALFDIESKVDPSQAWGEVKRQISIATFTVQAAAETLSEVA

SEQ ID NO: 2.

Example 2: Preparation of Mouse Anti-Human PSMA Monoclonal Antibody 2.1 Immunization and fusion

Mice were cross-immunized with the human PSMA-His protein (ACRO, PSA-H52H3) and LNCaP cells (ATCC, CRL-1740™). The amount of protein immunization was 25 μg, the cell immunization was at 5 × $10^6$ cells per immunization, and the immunization was performed once a week. After the mice were immunized 3 times, blood was taken to measure the titer of the antibody in the serum. The mice in which the antibody titer in the serum was high and was reaching a plateau were selected for spleen cell fusion. Spleen lymphocytes and myeloma cells, Sp2/0 cells (ATCC, CRL-8287™), were fused by following a PEG-mediated fusion procedure to give hybridoma cells. The fused hybridoma cells were resuspended in an MC semi-solid complete medium (RPMI-1640 medium containing 20% FBS, 1× HAT, 1× OPI, and 2% methylcellulose) at a density of 0.5-1 × $10^6$ cells/mL, and the suspension was aliquoted into 35-mm cell culture dishes and incubated at 37 °C with 5% $CO_2$ for 7-9 days. On day 7-9 after the fusion, according to the cell clone size, single cell clones were picked up into a 96-well cell culture plate to which 200 μL/well of HT complete medium (RPMI-1640 medium containing 20% FBS, 1× HT and 1× OPI) was added, cultured at 37 °C with 5% $CO_2$ for 3 days, and then detected.

### 2.2 Screening of hybridoma cells

Hybridoma culture supernatants were detected by a binding ELISA method according to the growth density of hybridoma cells. Hybridoma cells that had the good binding ability to huPSMA-CHO-K1 and cynoPSMA-CHO-K1 cells but did not bind to wild-type CHO-K1 cells were selected, expanded and cryopreserved in time, and subcloned two to three times until single cell clones were obtained.

### 2.3 Sequencing of hybridoma antibody

Monoclonal hybridoma cell strain mAb19-1 with high *in vitro* activity was selected. The antibody sequences therein were cloned, followed by humanization, recombinant expression, and activity evaluation.
The cloning of sequences from hybridoma was as follows: hybridoma cells at the logarithmic growth phase were collected, and the RNA was extracted using Trizol (Invitrogen, 15596-018) (according to the procedures in the kit instructions) and reverse transcribed (PrimeScript™ Reverse Transcriptase, Takara, cat # 2680A). The cDNA obtained by reverse transcription was amplified by PCR using the mouse Ig-Primer Set (Novagen, TB326 Rev.B 0503) and sent for sequencing by a sequencing company. The resulting antibody sequences are as follows:

mAb19-1 heavy chain variable region:

EVKLVESEGGLVQPGSSMKLSCTASGFTFSDYYMAWVRQVPERGLEWVANINYDGSSTYYLDSLKSRFIISRDNAKNILYLQMSSLKSEDTATYYCARGGDYWDAMDYWGQGTSVTVSS

SEQ ID NO: 3

mAb19-1 light chain variable region:

DIQMTQTTSSLSASLGDRVTISCRASQDISKYLNWYQQKPDGTVKLLIYYTSSLHSGVPSRFSGSGSGTDYSLTISNLEQEDIATYFCQQGNRLPRTFGGGTKLEIK

SEQ ID NO: 4

Note: The CDR regions determined according to the Kabat numbering scheme are underlined.

Table 2. CDR sequences of mAb19-1 antibody (according to the Kabat numbering scheme)

| Antibody | Name | Sequence | No. |
|---|---|---|---|
| mAb19-1 | HCDR1 | DYYMA | SEQ ID NO:5 |
| | HCDR2 | NINYDGSSTYYLDSLKS | SEQ ID NO:6 |
| | HCDR3 | GGDYWDAMDY | SEQ ID NO:7 |
| | LCDR1 | RASQDISKYLN | SEQ ID NO:8 |
| | LCDR2 | YTSSLHS | SEQ ID NO:9 |
| | LCDR3 | QQGNRLPRT | SEQ ID NO:10 |

Table 3. CDR sequences of mAb 19-1 antibody (according to the IMGT numbering scheme)

| Antibody | Name | Sequence | No. |
|---|---|---|---|
| mAb19-1 | HCDR1 | GFTFSDYY | SEQ ID NO:26 |
| | HCDR2 | INYDGSST | SEQ ID NO:27 |
| | HCDR3 | ARGGDYWDAMDY | SEQ ID NO:28 |
| | LCDR1 | QDISKY | SEQ ID NO:29 |
| | LCDR2 | YTS | SEQ ID NO:30 |
| | LCDR3 | QQGNRLPRT | SEQ ID NO:10 |

Table 4. CDR sequences of mAb 19-1 antibody (according to the Chothia numbering scheme)

| Antibody | Name | Sequence | No. |
|---|---|---|---|
| mAb19-1 | HCDR1 | GFTFSDY | SEQ ID NO:31 |
| | HCDR2 | NYDGSS | SEQ ID NO:32 |
| | HCDR3 | GGDYWDAMDY | SEQ ID NO:7 |
| | LCDR1 | RASQDISKYLN | SEQ ID NO:8 |
| | LCDR2 | YTSSLHS | SEQ ID NO:9 |
| | LCDR3 | QQGNRLPRT | SEQ ID NO:10 |

**Example 3: Humanization of Mouse Anti-Human PSMA Monoclonal Antibody**

By comparing the Kabat human antibody heavy and light chain variable region germline gene database with the MOE software, heavy and light chain variable region germline genes with high homology were selected as templates, and

CDRs of the mouse antibodies were grafted into corresponding human-derived templates to form variable region sequences in the following order: FR1-CDR1-FR2-CDR2-FR3-CDR3-FR4. Moreover, some amino acids in the FR regions were back-mutated and recombined with constant regions (illustratively, with a human IgG1 heavy chain constant region set forth in SEQ ID NO: 15 or 16 and a human κ light chain constant region set forth in SEQ ID NO: 17 or 18) to give the humanized antibodies.

The human germline light chain template of mAb19-1 antibody was IGKV1-39*01 and the human germline heavy chain template was IGHV3-7*01.

Table 5. Humanization design of mAb19-1 antibody

| VL | | VH | |
|---|---|---|---|
| VL1 | Graft (IGKV1-39*01) | VH1 | Graft (IGHV3-7*01) |
| VL2 | Graft (IGKV1-39*01) + F71Y | VH2 | Graft (IGHV3-7*01) + Q3K |

The variable region sequences of the humanized antibodies are as follows:

hu19-1 VH1

EVQLVESGGGLVQPGGSLRLSCAASGFTFSDYYMAWVRQAPGKGLEWVANIN
YDGSSTYYLDSLKSRFTISRDNAKNSLYLQMNSLRAEDTAVYYCARGGDYWDA
MDYWGQGTTVTVSS

SEQ ID NO: 11

hu19-1 VH2

EVKLVESGGGLVQPGGSLRLSCAASGFTFSDYYMAWVRQAPGKGLEWVANIN
YDGSSTYYLDSLKSRFTISRDNAKNSLYLQMNSLRAEDTAVYYCARGGDYWDA
MDYWGQGTTVTVSS

SEQ ID NO: 12

hu19-1 VL1

DIQMTQSPSSLSASVGDRVTITCRASQDISKYLNWYQQKPGKAPKLLIYYTSSLH
SGVPSRFSGSGSGTDFTLTISSLQPEDFATYYCQQGNRLPRTFGGGTKVEIK

SEQ ID NO: 13

hu19-1 VL2

DIQMTQSPSSLSASVGDRVTITCRASQDISKYLNWYQQKPGKAPKLLIYYTSSLH
SGVPSRFSGSGSGTDYTLTISSLQPEDFATYYCQQGNRLPRTFGGGTKVEIK

SEQ ID NO: 14

Note: The CDRs are underlined with a single line, and the back mutation positions are underlined with double lines.

The constant regions of the antibodies are as follows:

Human IgG1 heavy chain constant region

ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTC
PPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPE
NNYKTTPPVLDSDGSFFLYSKLTVDKSRWQQGNVFSCSVMHEALHNHYTQKSL
SLSPGK

SEQ ID NO: 15

Human IgG1 heavy chain constant region variant:

ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKS**S**DKTHTCP
PCPAPELLGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIEK
TISKAKGQPREPQVYTLPPSRDELTKNQVSLTCLVKGFYPSDIAVEWESNGQPEN
NYKTTPPVLDSDGSFFLYSKLTVDKSRWQQGNVFSCSVMHEALHNHYTQKSLS
LSPGK

SEQ ID NO: 16

κ chain constant region:

RTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC

SEQ ID NO: 17

κ chain constant region variant:

RTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGE**S**

SEQ ID NO: 18

Combinations of the heavy and light chain variable regions of exemplary humanized antibodies are as follows:

Table 6. Humanized antibodies of mAb19-1

| Variable region | VL1 | VL2 |
|---|---|---|
| VH1 | 19L1H1 | 19L2H1 |
| VH2 | 19L1H2 | 19L2H2 |

Note: 19L1H1 indicates that the antibody comprises the heavy chain variable region hu19-1VH1 and the light chain variable region hu19-1VL1; moreover, the sequence of the heavy chain constant region thereof is set forth in SEQ ID NO: 15, and the sequence of the light chain constant region is set forth in SEQ ID NO: 17, and so on.

The antibodies described above were separately cloned, expressed, and purified, and finally, the humanized antibody with good activity was selected by an affinity assay. The heavy and light chain amino acid sequences of an exemplary humanized antibody P19 are as follows:

P19 (19L2H2) heavy chain:

EVKLVESGGGLVQPGGSLRLSCAASGFTFSDYYMAWVRQAPGKGLEWVANIN
YDGSSTYYLDSLKSRFTISRDNAKNSLYLQMNSLRAEDTAVYYCARGGDYWDA
MDYWGQGTTVTVSS*ASTKGPSVFPLAPSSKSTSGGTAALGCLVKDYFPEPVTVSWN*
*SGALTSGVHTFPAVLQSSGLYSLSSVVTVPSSSLGTQTYICNVNHKPSNTKVDKKVEPK*
*SCDKTHTCPPCPAPELLGGPSVFLFPPKPKDTLMISRTPEVTCVVVDVSHEDPEVKF*
*NWYVDGVEVHNAKTKPREEQYNSTYRVVSVLTVLHQDWLNGKEYKCKVSNKALPAP*
*IEKTISKAKGQPREPQVYTLPPSRDELTKNQVSLTCLVKGFYPSDIAVEWESNGQPEN*
*NYKTTPPVLDSDGSFFLYSKLTVDKSRWQQGNVFSCSVMHEALHNHYTQKSLSLSPG*
*K*

SEQ ID NO: 19

P19 (19L2H2) light chain:

DIQMTQSPSSLSASVGDRVTITCRASQDISKYLNWYQQKPGKAPKLLIYYTSSLH
SGVPSRFSGSGSGTDYTLTISSLQPEDFATYYCQQGNRLPRTFGGGTKVEIK*RTVA*
*APSVFIFPPSDEQLKSGTASVVCLLNNFYPREAKVQWKVDNALQSGNSQESVTEQDS*
*KDSTYSLSSTLTLSKADYEKHKVYACEVTHQGLSSPVTKSFNRGEC*

Note: In the sequence, the variable regions are underlined, and the constant regions are italicized.

SEQ ID NO: 20.

A positive control antibody H-AB-P1 (fully known as AB-PG1-XG1-006, prepared with reference to WO2003034903A2) was used in the present disclosure to prepare an antibody-drug conjugate. The sequences are as follows:

H-AB-P1 heavy chain:

QVQLVESGGGVVQPGRSLRLSCAASGFAFSRYGMHWVRQAPGKGLEWVAVIW
YDGSNKYYADSVKGRFTISRDNSKNTQYLQMNSLRAEDTAVYYCARGGDFLYY
YYYGMDVWGQGTTVTVSS*ASTKGPSVFPLAPSSKSTSGGTAALGCLVKDYFPEPV*
*TVSWNSGALTSGVHTFPAVLQSSGLYSLSSVVTVPSSSLGTQTYICNVNHKPSNTKVDK*
*KVEPKSCDKTHTCPPCPAPELLGGPSVFLFPPKPKDTLMISRTPEVTCVVVDVSHED*
*PEVKFNWYVDGVEVHNAKTKPREEQYNSTYRVVSVLTVLHQDWLNGKEYKCKVSNK*
*ALPAPIEKTISKAKGQPREPQVYTLPPSRDELTKNQVSLTCLVKGFYPSDIAVEWESNG*
*QPENNYKTTPPVLDSDGSFFLYSKLTVDKSRWQQGNVFSCSVMHEALHNHYTQKSL*
*SLSPGK*

SEQ ID NO: 21

H-AB-P1 light chain:

DIQMTQSPSSLSASVGDRVTITCRASQGISNYLAWYQQKTGKVPKFLIYEASTLQSGVPSRFSGGGSGTDFTLTISSLQPEDVATYYCQNYNSAPFTFGPGTKVDIK*RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC*

SEQ ID NO: 22.

A negative control antibody hIgG1 was used in the present disclosure to prepare an antibody-drug conjugate. The sequences are as follows:

hIgG1 heavy chain:

QVQLVQSGAEVKKPGASVKVSCKASGYTFTNSWIGWFRQAPGQGLEWIGDIYPGGGYTNYNEIFKGKATMTADTSTNTAYMELSSLRSEDTAVYYCSRGIPGYAMDYWGQGTLVTVSS*ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK*

SEQ ID NO: 23

hIgG1 light chain:

DIQMTQSPSSLSASVGDRVTMSCKSSQSLLNSGDQKNYLTWYQQKPGKAPKLLIYWASTGESGVPSRFSGSGSGTDFTFTISSLQPEDIATYYCQNDYSYPWTFGQGTKVEIK*RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC*

SEQ ID NO: 24.

## II. Preparation of ADCs

Drug loading analysis of ADCs

The DAR values of the ADCs of the present disclosure were calculated by RP-HPLC (reversed-phase high performance liquid chromatography), specifically as follows:

### Determination method

A naked antibody and an ADC test sample (at a concentration of 1 mg/mL) were reduced with 4 μL of 0.25 M DDT (sigma) in a water bath at 37 °C for 1 h, and then transferred to an insert. Analysis was performed on a high performance liquid chromatograph Agilent 1200, with Agilent PLRP-S 1000A 8 μm 4.6 × 250 mm selected as the chromatography column, the column temperature at 80 °C, the DAD detector at wavelength 280 nm, the flow rate at 1 mL/min, and the injection volume at 40 μL. Comparisons were made to the spectra of the sample and the naked antibody to identify the locations of the light chain and heavy chain, and then integration was performed on the spectrum of the test sample to calculate the DAR value.

**Preparation of solutions**

1) 0.25 M DTT solution:
Example of preparation: DTT (5.78 mg) was weighed out and completely dissolved in purified water (150 μL) to give a 0.25 M DTT solution, and the solution was then stored at -20 °C.
2) Mobile phase A (0.1% TFA in water):
Example of preparation: Purified water (1000 mL) was measured out using a graduated cylinder, and TFA (1 mL) (sigma) was added. The solution was well mixed before use and was stored at 2-8 °C for 14 days.
3) Mobile phase B (0.1% TFA in acetonitrile):
Example of preparation: Acetonitrile (1000 mL) was measured out using a graduated cylinder, and TFA (1 mL) was added. The solution was well mixed before use and was stored at 2-8 °C for 14 days.

**Data analysis**

Comparisons were made to the spectra of the sample and the naked antibody to identify the locations of the light chain and heavy chain, and then integration was performed on the spectrum of the test sample to calculate the DAR value.
The calculation formula is as follows:

Table 7

| Name | Number of linked drugs |
|---|---|
| LC | 0 |
| LC+1 | 2 |
| HC | 0 |
| HC+1 | 2 |
| HC+2 | 4 |
| HC+3 | 6 |

$$\text{Total LC peak area} = \text{LC peak area} + \text{LC+1 peak area}$$

Total HC peak area = HC peak area + HC+1 peak area + HC+2 peak area + HC+3 peak area

$$\text{LC DAR} = \Sigma(\text{number of linked drugs} \times \text{percent peak area})/\text{total LC peak area}$$

$$\text{HC DAR} = \Sigma(\text{number of linked drugs} \times \text{percent peak area})/\text{total HC peak area}$$

$$\text{DAR} = \text{LC DAR} + \text{HC DAR}.$$

<u>Preparation examples of PSMA antibody-drug conjugate P19-9-A with different DAR values</u>

The following examples are the preparation processes of related ADCs of the present disclosure. In **Example 4-1, Example 4-2, Example 4-3,** and **Example 4-4,** the antibody P19 was subjected to a coupling reaction with the drug **9-A** having a linker unit via a sulfhydryl group on cysteine to prepare an antibody-drug conjugate; in **Example 4-5,** the antibody H-AB-P1 was subjected to a coupling reaction with the drug **9-A** having a linker unit via a sulfhydryl group on cysteine to prepare a control ADC: **H-AB-P1-9-A** (hereinafter referred to as ADC-5); in **Example 4-6,** the control antibody hIgG1 was subjected to a coupling reaction with the drug **9-A** having a linker unit via a sulfhydryl group on cysteine to prepare a control ADC: **hIgG1-9-A.**
By adjusting the ratio of antibody to drug, the reaction scale, and other conditions, the antibody-drug conjugate with different DAR values (n), preferably DAR values of 1 to 8, more preferably 3 to 8, and most preferably 5 to 8, could be

obtained.

(I) Preparation of antibody-drug conjugate P19-9-A with different DAR values

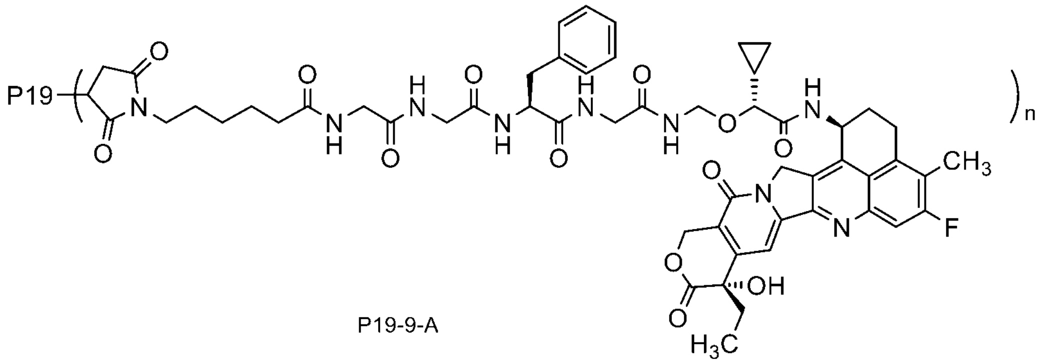

P19-9-A

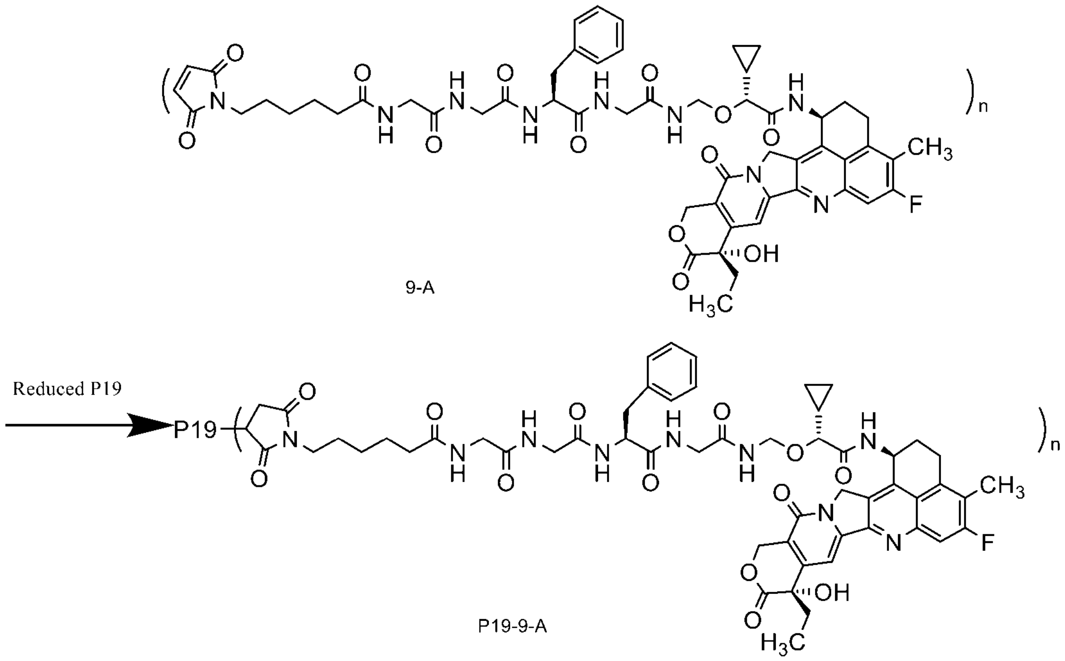


9-A

P19-9-A

**Example 4-1: ADC-1**

To an aqueous buffer solution of the antibody **P19** in PBS (0.05 M aqueous PBS buffer solution at pH 6.5; 10.0 mg/mL, 14 mL, 946 nM) was added a prepared aqueous solution of tris(2-carboxyethyl)phosphine hydrochloride (TCEP.HCl) (10 mM, 312 μL, 3120 nM) at 37 °C. The mixture was shaken on a water bath shaker at 37 °C for 3 h, and then the reaction was stopped. The reaction mixture was cooled to 25 °C in a water bath.

The compound 9-A (prepared with reference to 9-A in Example 9 on pages 58-60 of WO2020063676A1, 12.19 mg, 11.35 μM) was dissolved in a proper amount of dimethyl sulfoxide, and the resulting solution was added to the reaction mixture described above. The mixture was shaken on a water bath shaker at 25 °C for 3 h, and then the reaction was stopped. The reaction mixture was desalted and purified through a Sephadex G25 gel column (manufacturer: cytiva; Cat. # 17003201; elution phase: 0.05 M aqueous PBS buffer solution at pH 6.5, containing 0.001 M EDTA) to give an exemplary product ADC-**1** of the conjugate of formula P19-9-A in a PBS buffer (3.48 mg/mL, 35.3 mL), which was refrigerated at 4 °C.

Mean calculated by RP-HPLC: n = 5.55.

**Example 4-2: ADC-2**

To an aqueous buffer solution of the antibody **P19** in PBS (0.05 M aqueous PBS buffer solution at pH 6.5; 10.0 mg/mL, 40 mL, 2.703 μM) was added a prepared aqueous solution of tris(2-carboxyethyl)phosphine hydrochloride

(TCEP.HCl) (10 mM, 1.621 mL, 16.21 μM) at 37 °C. The mixture was shaken on a water bath shaker at 37 °C for 3 h, and then the reaction was stopped. The reaction mixture was cooled to 25 °C in a water bath. The compound **9-A** (43.54 mg, 40.54 μM) was dissolved in a proper amount of dimethyl sulfoxide, and the resulting solution was added to the reaction mixture described above. The mixture was shaken on a water bath shaker at 25 °C for 3 h, and then the reaction was stopped. The reaction mixture was desalted and purified through a Sephadex G25 gel column (elution phase: 0.05 M aqueous PBS buffer solution at pH 6.5, containing 0.001 M EDTA) to give an exemplary product **ADC-2** of the conjugate of formula P19-9-A in a PBS buffer (4.25 mg/mL, 80.3 mL), which was refrigerated at 4 °C.

Mean calculated by RP-HPLC: n = **7.41.**

**Example 4-3: ADC-3**

To an aqueous buffer solution of the antibody **P19** in PBS (0.05 M aqueous PBS buffer solution at pH 6.5; 10.0 mg/mL, 1.5 mL, 101.3 nM) was added a prepared aqueous solution of tris(2-carboxyethyl)phosphine hydrochloride (TCEP.HCl) (10 mM, 32.4 μL, 324 nM) at 37 °C. The mixture was shaken on a water bath shaker at 37 °C for 3 h, and then the reaction was stopped. The reaction mixture was cooled to 25 °C in a water bath. The compound **9-A** (1.31 mg, 1215 nM) was dissolved in a proper amount of dimethyl sulfoxide, and the resulting solution was added to the reaction mixture described above. The mixture was shaken on a water bath shaker at 25 °C for 3 h, and then the reaction was stopped. The reaction mixture was desalted and purified through a Sephadex G25 gel column (elution phase: 0.05 M aqueous PBS buffer solution at pH 6.5, containing 0.001 M EDTA) to give an exemplary product **ADC-3** of the conjugate of formula P19-9-A in a PBS buffer (0.85 mg/mL, 12.5 mL), which was refrigerated at 4 °C.

Mean calculated by RP-HPLC: n = 5.5.

**Example 4-4: ADC-4**

To an aqueous buffer solution of the antibody **P19** in PBS (0.05 M aqueous PBS buffer solution at pH 6.5; 10.0 mg/mL, 1.5 mL, 101.3 nM) was added a prepared aqueous solution of tris(2-carboxyethyl)phosphine hydrochloride (TCEP.HCl) (10 mM, 60.8 μL, 608 nM) at 37 °C. The mixture was shaken on a water bath shaker at 37 °C for 3 h, and then the reaction was stopped. The reaction mixture was cooled to 25 °C in a water bath. The compound **9-A** (1.63 mg, 1520 nM) was dissolved in a proper amount of dimethyl sulfoxide, and the resulting solution was added to the reaction mixture described above. The mixture was shaken on a water bath shaker at 25 °C for 3 h, and then the reaction was stopped. The reaction mixture was desalted and purified through a Sephadex G25 gel column (elution phase: 0.05 M aqueous PBS buffer solution at pH 6.5, containing 0.001 M EDTA) to give an exemplary product **ADC-4** of the conjugate of formula P19-9-A in a PBS buffer (0.89 mg/mL, 12.9 mL), which was refrigerated at 4 °C.

Mean calculated by RP-HPLC: n = **7.34.**

(II) Preparation of control antibody-drug conjugate H-AB-P1-9-A

**Example 4-5: ADC-5**

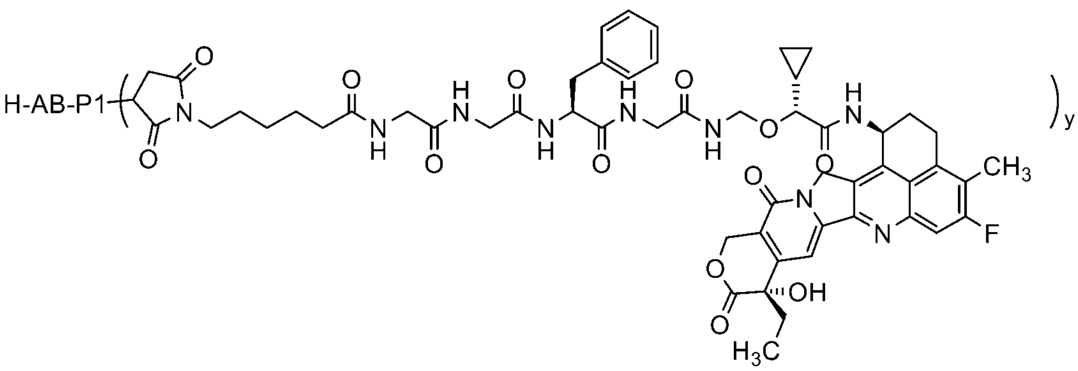

H-AB-P1-9-A

To an aqueous buffer solution of the antibody **H-AB-P1** in PBS (0.05 M aqueous PBS buffer solution at pH 6.5; 10.0 mg/mL, 1.5 mL, 101 nmol) was added a prepared aqueous solution of tris(2-carboxyethyl)phosphine (TCEP.HCl) (10 mM, 60.8 μL, 608 nmol) at 37 °C. The mixture was shaken on a water bath shaker at 37 °C for 3 h, and then the reaction was

stopped. The reaction mixture was cooled to 25 °C in a water bath.

The compound **9-A** (1.63 mg, 1.517 μmol) was dissolved in a proper amount (75 μL) of dimethyl sulfoxide, and the resulting solution was added to the reaction mixture described above. The mixture was shaken on a water bath shaker at 25 °C for 3 h, and then the reaction was stopped. The reaction mixture was desalted and purified through a Sephadex G25 gel column (elution phase: 0.05 M aqueous PBS buffer solution at pH 6.5, containing 0.001 M EDTA) to give the title product **ADC-5** in a PBS buffer, which was refrigerated at 4 °C.

Mean calculated by RP-HPLC: **y = 7.25.**

(III) Preparation of control antibody-drug conjugate hIgG1-9-A

**Example 4-6: hIgG1-9-A**

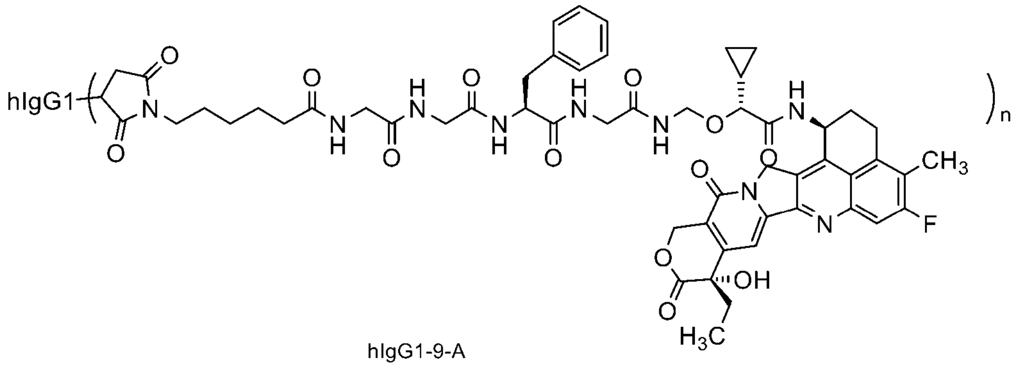

hIgG1-9-A

To an aqueous buffer solution of the antibody **hIgG1** in PBS (0.05 M aqueous PBS buffer solution at pH 6.5; 10.0 mg/mL, 4 mL, 270.2 nM) was added a prepared aqueous solution of tris(2-carboxyethyl)phosphine hydrochloride (TCEP.HCl) (10 mM, 162.2 μL, 1622 nM) at 37 °C. The mixture was shaken on a water bath shaker at 37 °C for 3 h, and then the reaction was stopped. The reaction mixture was cooled to 25 °C in a water bath. The compound **9-A** (4.35 mg, 4050 nM) was dissolved in a proper amount of dimethyl sulfoxide, and the resulting solution was added to the reaction mixture described above. The mixture was shaken on a water bath shaker at 25 °C for 3 h, and then the reaction was stopped. The reaction mixture was desalted and purified through a Sephadex G25 gel column (elution phase: 0.05 M aqueous PBS buffer solution at pH 6.5, containing 0.001 M EDTA) to give the title product **hIgG1-9-A** in a PBS buffer (1.95 mg/mL, 16.7 mL), which was refrigerated at 4 °C.

Mean calculated by RP-HPLC: n = **7.60.**

**Verification of activity of antibody and ADCs of the present disclosure with biochemical test methods**

**Test Example 1: Antibody Cell-Level FACS Binding Assay**

In this assay, fluorescence signals of the antibody on the cell surface were detected, and the binding of the antibody was evaluated according to the intensity of the fluorescence signals.

Cells LNCaP (human prostate cancer cell, ATCC, CRL-1740), 22RV1 (human prostate cancer cell, ATCC, CRL-2505), and PC-3 (human prostate cancer cell, ATCC, CRL-1435) were suspended in a FACS buffer (2% fetal bovine serum (Gibco, 10099141) and PBS at pH 7.4) to prepare cell suspensions at 1 × $10^6$ cells/mL, and the suspensions were then added to a 96-well round-bottom plate at 100 μL/well. After centrifugation and removal of the supernatant, the test antibody that was diluted with the FACS buffer to different concentrations was added at 50 μL/well. The plate was incubated in the dark in a refrigerator at 4 °C for 1 h. The plate was washed 3 times with the FACS buffer by centrifugation at 500 g, and a FITC-labeled goat anti-human IgG (H + L) secondary antibody (Jackson Immuno Research, 109-095-003) at a working concentration was added. The plate was incubated in the dark in a refrigerator at 4 °C for 40 min. The plate was washed 3 times with the FACS buffer by centrifugation at 500 g and tested on a BD FACSCantoII flow cytometer for geometric mean fluorescence intensity, and the $EC_{50}$ values for the binding of the antibody to the cells expressing PSMA were calculated. The results are shown in Table 8 below and FIGs. 1A, 1B, and 1C.

Table 8. *In vitro* binding activity of antibody to cells with differential expression levels of PSMA ($EC_{50}$, nM)

| | LNCaP (+++) | 22RV1 (+) | PC-3 (-) |
|---|---|---|---|
| P19 | 2.094 | 0.6366 | No binding |
| Note: + represents the expression level of PSMA on the cells, and more + represents a higher expression level; - means that PSMA is not expressed. | | | |

The results show that P19 had good binding activity to tumor cells expressing PSMA.

**Test Example 2: Detection of Affinity of Antibody by Biacore**

The IgG antibody was subjected to affinity capture by a Protein A biosensor chip (Cat. # 29127556, GE), and then human PSMA-His (ACRO, PSA-H52H3), monkey PSMA-His (ACRO, PSA-C5247), and mouse PSMA-His (ACRO, PSA-M5245) antigens that were diluted with an HBS-EP buffer at pH 7.4 (Cat. # BR-1001-88, GE) to a series of concentrations were allowed to flow over the surface of the chip. The antigen-antibody binding kinetics was tracked for 3 min, and the dissociation kinetics was tracked for 10 min. Reaction signals were detected in real time using a Biacore T200 instrument (GE) to give binding and dissociation curves. After dissociation was complete in each cycle, the biosensor chip was washed with 10 mM Gly-HCl at pH 1.5 (Cat. # BR-1003-54, GE) for regeneration. The data obtained were analyzed with BIAevaluation software of GE using a 1:1 (Langmuir) binding model. The results of the determined ka (kon), kd (koff), and KD values are shown in Table 9 below.

Table 9. Affinity of antibody for PSMA proteins

| | **Human PSMA-his** | | | **Monkey PSMA-his** | **Mouse PSMA-his** |
|---|---|---|---|---|---|
| **Antibody** | **KD (M)** | **kon (1/Ms)** | **kdis (1/s)** | **KD (M)** | **KD (M)** |
| P19 | 1.30E-09 | 6.11E+04 | 7.92E-05 | 5.08E-09 | No binding |

The results show that the antibody disclosed herein had relatively high affinity for human and cynomolgus monkey PSMA, but did not bind to mouse PSMA.

**Test Example 3: Antibody DT3C Endocytosis Assay**

The purpose of this assay is that the activated DT kills cells after the DT3C protein enters the cells, indirectly reflecting endocytosis of the PSMA antibody. The *in vitro* endocytic activity of the antibodies was evaluated according to $IC_{50}$ and maximal killing values.

DT3C is a recombinantly expressed fusion protein formed by fusing fragment A of diphtheria toxin (toxin portion only) and fragment 3C of group G streptococcus (IgG binding portion). The protein has a high affinity for the IgG portion of an antibody. It enters cells together with the IgG portion when the antibody is endocytosed, and releases toxic DT under the action of intracellular furin protease. The DT can inhibit the activity of EF2-ADP ribosylation, block the protein translation process, and finally cause cell death. DT3C molecules outside the cell have no cell killing activity. The endocytic activity of the antibodies can be evaluated based on cell killing.

A suspension of LNCaP cells (ATCC, CRL-1740) at a cell density of $5 \times 10^4$ cells/mL was prepared with a fresh cell medium containing 20% low IgG FBS and added to a cell culture plate at 50 μL/well. The plate was incubated at 37 °C with 5% $CO_2$ for 16 h. DT3C (Sigma, Cat. # D0564) was diluted to 1.6 μM with serum-free medium, and the antibodies were diluted to 133.2 nM with serum-free medium. DT3C (80 μL) and the antibody solution (80 μL) were mixed (1:1, v/v) and incubated at room temperature for 30 min. The molar concentration of DT3C was 6 times that of the antibodies.

The DT3C-antibody mixture was serially diluted 4-fold with serum-free medium to 8 concentrations. The 9th point was a pure medium. hIgG1 was an IgG1 isotype negative control group. The diluted mixture (50 μL) was added to cells (50 μL) and incubated in an incubator for three days. To each well, 50 μL of CTG was added. The plate was incubated in the dark at room temperature for 10 min. A white membrane was attached to the bottom of the cell culture plate. The plate was placed on a microplate reader Victor 3, and chemiluminescence readings were taken. The results are shown in Table 10 below.

Table 10. *In vitro* endocytic activity of antibodies

| Antibody | $IC_{50}$ (nM) | Imax (%) |
|---|---|---|
| P19 | 0.25 | 88.0 |
| H-AB-P1 control | 2.71 | 57.7 |
| hIgG1 control | No endocytosis | No endocytosis |

The results show that P19 could be endocytosed by LNCaP cells expressing PSMA, and the endocytic activity of P19 was significantly superior to that of the control antibody H-AB-P1.

**Test Example 4: *In Vitro* Cell Binding Assay of ADCs**

In this assay, fluorescence signals of the antibody on the cell surface were detected, and the binding of the antibody was evaluated according to the intensity of the fluorescence signals. The prepared ADC-1, ADC-2, control ADC hIgG1-9-A, and antibody P19 were used together for the *in vitro* binding assay.

The serially diluted ADC-1, ADC-2, control ADC hIgG1-9-A, and antibody P19 were separately incubated with $1 \times 10^5$ cells (LNCaP/CRL-1740, 22Rv1/CRL-2505, PC-3/CRL-1435) at 4 °C for 60 min, and then excess ADCs or antibodies were washed off. The cells were incubated with a FITC-labeled goat anti-human IgG (H + L) secondary antibody (Jackson Immuno Research, 109-095-003) at 4 °C for 30 min, and then excess antibodies were washed off. The fluorescence signals on the cell surface were read using BD CantoII. The results are shown in Table 11.

Table 11. *In vitro* cell binding activity of ADCs ($EC_{50}$, nM)

| | LNCaP (+++) | 22RV1 (+) | PC-3 (-) |
|---|---|---|---|
| ADC-1 | 0.4419 | 0.1136 | No binding |
| ADC-2 | 0.3074 | 0.08119 | No binding |
| hIgG1-9-A | No binding | No binding | No binding |
| P19 | 0.3052 | 0.0719 | No binding |
| Note: + represents the expression level of PSMA on the cells, and more + represents a higher expression level; - means that PSMA is not expressed. | | | |

The results show that the ADC-1, ADC-2, control ADC hIgG1-9-A, and antibody P19 had correspondingly strong or weak binding ability to cells with different expression levels of the PSMA antigen. The binding of the P19 antibody to the tumor cells expressing PSMA did not change significantly after conjugation of the antibody into ADCs.

**Test Example 5: Cell Activity of ADC Molecules**

The purpose of this assay is to measure the killing effects of the compound 2-B and ADC molecules thereof on cells expressing PSMA and evaluate the *in vitro* activity of PSMA-ADC according to $IC_{50}$ and $I_{max}$.

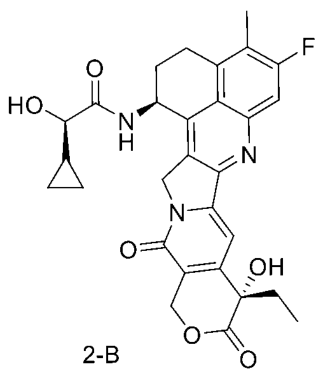

2-B

LNCaP (ATCC, CRL-1740), 22RV1 (ATCC, CRL-2505), and PC-3 (ATCC, CRL-1435) cells were digested with trypsin, neutralized with a fresh medium, centrifuged at 1000 rpm, and then resuspended in a medium. After the cells were

counted, the density of the cell suspensions was adjusted to 1.48 × $10^4$ cells/mL, and the cells were plated onto a 96-well plate. The adjusted cell suspension (135 μL) was added to each well with 1000 cells/well. Only the middle 60 wells were used for plating, and six wells in the twelfth column were taken as pure medium controls without cells. An equal volume of PBS was added to the other wells. The cells were incubated at 37 °C with 5% $CO_2$ for 16 h.

The test ADCs (ADC-1, ADC-2, and control ADC: hIgG1-9-A) and antibody P19 were prepared into an initial concentration (1000 nM) and diluted in a gradient dilution ratio of 1:5 with PBS to give 9 concentration points. Then 20 μL of the dilution was added to the cell plate described above, and the plate was incubated in an incubator (37 °C, 5% CO2) for 5 days. The compound 2-B (i.e., free toxin of 9-A, prepared with reference to 2-B in Example 2 on page 50 of WO2020063676A1) was diluted into a stock solution with DSMO, followed by dilution with PBS to a starting concentration of 100 nM. Subsequently, 9-point serial dilutions at a 1:3 ratio were performed with PBS. Then 1 μL of the dilution was added to 20 μL of RPMI-1640, and the mixture was transferred to a 96-well cell culture plate. The plate was incubated in an incubator (37 °C, 5% CO2) for 5 days. In the 96-well cell culture plate, 80 μL of the CellTiter-Glo reagent was added to each well, and the plate was left to stand in the dark at room temperature for 10-15 min. The chemiluminescence signal values were read on Victor3, and the data were processed using GraphPad software.

On the fifth day, the culture plate was taken out and allowed to return to room temperature. The CellTiter-Glo assay solution (75 μL) was added to each well, and the plate was left to stand in the dark at room temperature for 10 min. A Backseal membrane was attached to the bottom of the cell culture plate. The plate was placed on a microplate reader Victor 3, and luminescence signal values were read. Curve fitting was performed according to the log values of concentrations of the test compounds to the corresponding signal values using Graphpad Prism software, and IC50 values were calculated. The maximum value of killing was defined as the maximum killing rate (Imax) of the compound. The killing rate was calculated as follows:

**killing** rate (%) = $(RLU_{\text{no drug control reading}} - RLU_{\text{sample reading}})/(RLU_{\text{no drug control reading}} - RLU_{\text{vehicle control reading}}) \times 100\%$

The results are shown in Table 12.

Table 12. Killing effects of ADCs on LNCaP, 22RV1, and PC-3 cells

| Sample name | LNCaP (+++) | | 22RV1 (+) | | PC-3 (-) | |
|---|---|---|---|---|---|---|
| | $IC_{50}$ (nM) | Imax (%) | $IC_{50}$ (nM) | Imax (%) | $IC_{50}$ (nM) | Imax (%) |
| ADC-1 | 8.522 | 88.52 | 29.54 | 92.91 | 219.7 | 80.31 |
| ADC-2 | 2.826 | 94.91 | 6.925 | 98.03 | 119.1 | 90.73 |
| hIgG 1-9-A | 112.5 | 88.12 | 110.7 | 93.59 | 282.6 | 85.02 |
| P19 | No killing | 18.57 | No killing | 6.64 | No killing | 1.02 |
| Compound 2-B | 0.5347 | 97.13 | 0.5336 | 98.07 | 1.541 | 92.78 |
| Note: + represents the expression level of PSMA on the cells, and more + represents a higher expression level; - means that PSMA is not expressed. | | | | | | |

The results show that PSMA-ADC had a significant killing effect on the cells with a high PSMA expression level, a significantly reduced killing effect on the cells with a low PSMA expression level, and almost no killing on the cells not expressing PSMA, that is, the ADC molecules in the present disclosure have selective killing effects on the cells. Whereas the cytotoxic compound 2-B (i.e., the free toxin of 9-A) had no selectivity in killing cells.

### Test Example 6: *In Vivo* Efficacy Evaluation of ADC Molecules in CDX Model with Moderate Expression of PSMA

The 22RV1 cells (200 μL) were inoculated subcutaneously into the right flank of 50 male NU/NU nude mice at 2 × $10^6$ cells/mouse. When the tumor volume of tumor-bearing mice reached about 188 $mm^3$, mice with too large or too small tumor volume and with too small body weight were excluded. 35 mice were selected and randomized into 5 groups of 7 mice. The day of grouping was defined as day 0 of the experiment, and each drug was intraperitoneally injected on day 0 (hereinafter referred to as D0), day 7 (hereinafter referred to as D7), and day 14 (hereinafter referred to as D14) for a total of 3 times.

The tumor volume and body weight were measured twice a week, and the data were recorded. The experimental

data were expressed as mean ± standard error (SEM) and plotted using GraphPad Prism software, and statistical analysis was performed.

The tumor volume (V) was calculated as follows: V = 1/2 × a × $b^2$, where a and b represent length and width, respectively.

Relative tumor proliferation rate T/C (%) = (T - T0)/(C - C0) × 100%, where T and C are the tumor volumes of animals at the end of the experiment in the treatment group and control group, respectively; T0 and C0 are the tumor volumes of animals at the beginning of the experiment.

$$\text{Tumor growth inhibition rate TGI (\%)} = 1 - \text{T/C (\%)}.$$

The results are shown in Table 13.

Table 13. Efficacy of ADC molecules on NU/NU nude mouse 22RV1 tumor

| Group | Administration time | Mean tumor volume ($mm^3$) | | Mean tumor volume ($mm^3$) | | Tumor growth inhibition rate TGI (%) |
|---|---|---|---|---|---|---|
| | | D0 | SEM | D17 | SEM | D17 |
| Vehicle control | D0, D7, D14 | 188.6 | 15.6 | 1725.2 | 257.8 | 0.00% |
| ADC-3 3mpk | D0, D7, D14 | 183.9 | 13.6 | 509.2 | 35.6 | 78.83% |
| ADC-3 1mpk | D0, D7, D14 | 187.5 | 16.8 | 900.6 | 111.8 | 53.59% |
| ADC-4 3mpk | D0, D7, D14 | 185.9 | 17.5 | 501.6 | 54.8 | 79.45% |
| ADC-4 1mpk | D0, D7, D14 | 185.3 | 13.3 | 763.1 | 99.9 | 62.40% |

The results show that ADC-3 and ADC-4 both had significant tumor inhibition effects in the mouse 22RV1 tumor model. The tumor inhibition effect was positively correlated with the administration dose, and the tumor inhibition effect of the ADC with a high DAR value (n = 7.34) was superior to that of the ADC with a low DAR value (n = 5.5). The body weight of the mice did not change significantly.

**Test Example 7: PK Evaluation of ADC Molecules**

1. Study of single-dose pharmacokinetics of ADC molecules in adult SD rats.

Adult SD rats (Zhejiang Vital River Laboratory Animal Technology Co., Ltd.) were randomly grouped with 4 rats for each ADC molecule, and the ADC molecule was injected intravenously at 3 mpk (n = 4 rats/group). Sera were collected at 0.083 h, 8 h, 24 h, 48 h, 96 h, 168 h, 240 h, 336 h, 504 h, and 672 h after intravenous injection in the rats for bioanalytical measurements. The rat serum samples were determined using an HTRF method, and the quantitative analysis of the content of the test samples was performed by a four-parameter model curve of the standard substance. Pharmacokinetic parameters were analyzed using a standard non-compartmental model of WinNonlin software (6.1). The results are shown in Table 14 and FIG. 2.

Table 14. PK of ADC molecules in SD rats

| | | ADC-4 | ADC-5 |
|---|---|---|---|
| Route of administration | | 3 mg/kg, i.v., single dose | 3 mg/kg, i.v., single dose |
| $T_{1/2}$ (day) | TAb | 8.2 ± 0.5 | 5.3 ± 0.3 |
| | Intact ADC | 6.9 ± 0.1 | 4.8 ± 0.3 |
| AUC 0-t (μg/mL*h) | TAb | 5304.28 | 2538.30 |
| | Intact ADC | 5239.00 | 2415.28 |
| | Intact ADC/TAb | 99% | 95% |
| Note: i.v. represents intravenous injection; TAb represents total antibody; Intact ADC represents the intact ADC. This applies hereinafter. | | | |

The half-lives of the total antibody and intact ADC of ADC-4 in rats were 8.2±0.5 days and 6.9±0.1 days, respectively, at an administration dose of 3 mpk by intravenous injection. Whereas the half-lives of the total antibody and intact ADC of ADC-5 in rats were 5.3±0.3 days and 4.8±0.3 days, respectively. Apparently, ADC-4 had a significantly better half-life in rats than that of ADC-5 and was more stable *in vivo.*

2. Study of single-dose pharmacokinetics of ADC molecules in adult male cynomolgus monkeys.

Cynomolgus monkeys (Suzhou Guochen Biotechnology Co., Ltd.) were grouped with 3 cynomolgus monkeys for each ADC molecule. After the ADC molecule was injected intravenously at 10 mpk (n = 3 cynomolgus monkeys/group), sera were collected at 0.083 h, 1 h, 6 h, 12 h, 24 h, 48 h, 96 h, 168 h, 240 h, 336 h, 504 h, and 672 h for bioanalytical measurements. The cynomolgus monkey serum samples were determined using an HTRF method, and the quantitative analysis of the content of the test samples was performed by a four-parameter model curve of the standard substance. Pharmacokinetic parameters were analyzed using a standard non-compartmental model of WinNonlin software (6.1). The results are shown in Table 15 and FIG. 3.

Table 15. PK of ADC molecules in cynomolgus monkeys

| | | ADC-2 |
|---|---|---|
| Route of administration | | 10 mg/kg, i.v., single dose |
| $T_{1/2}$ (day) | TAb | 6.3 ± **1.3** |
| | Intact ADC | 5.7 ± .0.9 |
| AUC 0-t (μg/mL*h) | TAb | 18691 |
| | Intact ADC | 19078 |
| | Intact ADC/TAb | 102% |

The half-lives of the total antibody and intact ADC of ADC-2 in cynomolgus monkeys were 6.3±1.3 days and 5.7 ±0.9 days, respectively, at an administration dose of 10 mpk by intravenous injection. The PK of ADC-2 in the cynomolgus monkeys is normal, and the stability is good.

## Claims

**1.** An antibody-drug conjugate of general formula Pc-L-Y-D or a pharmaceutically acceptable salt thereof:

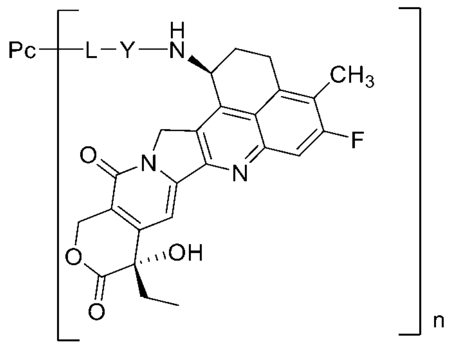

Pc-L-Y-D

wherein:
Pc is an anti-PSMA antibody or an antigen-binding fragment thereof, comprising a heavy chain variable region and a light chain variable region, wherein:

the anti-PSMA antibody comprises:

1) a HCDR1, a HCDR2, and a HCDR3 comprised in a heavy chain variable region set forth in SEQ ID NO: 3; and 2) a LCDR1, a LCDR2, and a LCDR3 comprised in a light chain variable region set forth in SEQ ID NO: 4;

preferably, the anti-PSMA antibody comprises a heavy chain variable region and a light chain variable region, wherein:

a. the heavy chain variable region comprises a HCDR1, a HCDR2, and a HCDR3 set forth in SEQ ID NO: 5, SEQ ID NO: 6, and SEQ ID NO: 7, respectively; the light chain variable region comprises a LCDR1, a LCDR2, and a LCDR3 set forth in SEQ ID NO: 8, SEQ ID NO: 9, and SEQ ID NO: 10, respectively, wherein the CDR regions are determined according to the Kabat numbering scheme; or
b. the heavy chain variable region comprises a HCDR1, a HCDR2, and a HCDR3 set forth in SEQ ID NO: 26, SEQ ID NO: 27, and SEQ ID NO: 28, respectively; the light chain variable region comprises a LCDR1, a LCDR2, and a LCDR3 set forth in SEQ ID NO: 29, SEQ ID NO: 30, and SEQ ID NO: 10, respectively, wherein the CDR regions are determined according to the IMGT numbering scheme; or
c. the heavy chain variable region comprises a HCDR1, a HCDR2, and a HCDR3 set forth in SEQ ID NO: 31, SEQ ID NO: 32, and SEQ ID NO: 7, respectively; the light chain variable region comprises a LCDR1, a LCDR2, and a LCDR3 set forth in SEQ ID NO: 8, SEQ ID NO: 9, and SEQ ID NO: 10, respectively, wherein the CDR regions are determined according to the Chothia numbering scheme;

Y is -O-C$R^1R^2$-C(O)-, wherein $R^1$ is $C_{1-6}$ haloalkyl or $C_{3-6}$ cycloalkyl; $R^2$ is selected from the group consisting of a hydrogen atom, $C_{1-6}$ haloalkyl, and $C_{3-6}$ cycloalkyl; or, $R^1$ and $R^2$, together with the carbon atom to which they are attached, form $C_{3-6}$ cycloalkyl;
L is a linker unit;
n is a decimal or an integer from 1 to 10.

**2.** The antibody-drug conjugate of general formula Pc-L-Y-D or the pharmaceutically acceptable salt thereof according to claim 1, wherein the anti-PSMA antibody is a mouse antibody, a chimeric antibody, a humanized antibody, or a human antibody.

**3.** The antibody-drug conjugate of general formula Pc-L-Y-D or the pharmaceutically acceptable salt thereof according to claim 1 or 2, wherein the anti-PSMA antibody or the antigen-binding fragment thereof comprises:

a heavy chain variable region set forth in SEQ ID NO: 11 or SEQ ID NO: 12, and
a light chain variable region set forth in SEQ ID NO: 13 or SEQ ID NO: 14;
preferably, the anti-PSMA antibody or the antigen-binding fragment thereof comprises:

a heavy chain variable region set forth in SEQ ID NO: 12, and
a light chain variable region set forth in SEQ ID NO: 14.

**4.** The antibody-drug conjugate of general formula Pc-L-Y-D or the pharmaceutically acceptable salt thereof according to any one of claims 1 to 3, wherein the anti-PSMA antibody comprises a heavy chain constant region and a light chain constant region; preferably, the heavy chain constant region is selected from the group consisting of human IgG1, IgG2, IgG3, and IgG4 constant regions and conventional variants thereof, and the light chain constant region is selected from the group consisting of human antibody $\kappa$ and $\lambda$ chain constant regions and conventional variants thereof;

more preferably, the heavy chain constant region is set forth in SEQ ID NO: 15 or SEQ ID NO: 16, and the light chain constant region is set forth in SEQ ID NO: 17 or SEQ ID NO: 18;
most preferably, the heavy chain constant region is set forth in SEQ ID NO: 15, and the light chain constant region is set forth in SEQ ID NO: 17.

**5.** The antibody-drug conjugate of general formula Pc-L-Y-D or the pharmaceutically acceptable salt thereof according to any one of claims 1 to 4, wherein the anti-PSMA antibody comprises a heavy chain set forth in SEQ ID NO: 19 and a light chain set forth in SEQ ID NO: 20.

**6.** The antibody-drug conjugate of general formula Pc-L-Y-D or the pharmaceutically acceptable salt thereof according to any one of claims 1 to 5, wherein n is a decimal or an integer from 1 to 8, preferably from 3 to 8, and more preferably from 5 to 8.

**7.** The antibody-drug conjugate of general formula Pc-L-Y-D or the pharmaceutically acceptable salt thereof according to any one of claims 1 to 6, wherein Y is selected from the group consisting of:

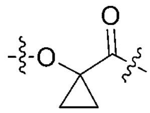, 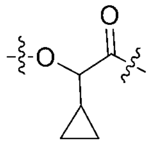, 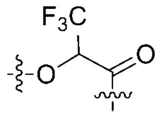, 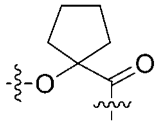, and 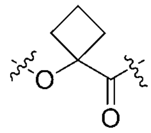;

wherein the O-terminus of Y is attached to L.

**8.** The antibody-drug conjugate of general formula Pc-L-Y-D or the pharmaceutically acceptable salt thereof according to any one of claims 1 to 7, wherein the linker unit -L-is $-L^1-L^2-L^3-$, wherein

$L^1$ is selected from the group consisting of -(succinimid-3-yl-*N*)-W-C(O)- and -C(O)-W-C(O)-, wherein W is selected from the group consisting of $C_{1-6}$ alkyl and $C_{1-6}$ alkyl-$C_{3-6}$ cycloalkyl;
$L^2$ is a peptide residue consisting of 2 to 7 amino acid residues, wherein the amino acid residues are selected from the group consisting of amino acid residues formed from amino acids of phenylalanine, glycine, valine, lysine, citrulline, serine, glutamic acid, and aspartic acid;
$L^3$ is selected from the group consisting of $-NR^3(CR^4R^5)_t-$, $-C(O)NR^3$, $-C(O)NR^3(CH_2)_t-$, and a chemical bond, wherein t is an integer from 1 to 6;
$R^3$, $R^4$, and $R^5$ are identical or different and are each independently selected from the group consisting of a hydrogen atom, halogen, $C_{1-6}$ alkyl, $C_{1-6}$ haloalkyl, $C_{1-6}$ deuterated alkyl, and $C_{1-6}$ hydroxyalkyl;
wherein the $L^1$ terminus is attached to Pc, and the $L^3$ terminus is attached to a drug; preferably,
$L^1$ is

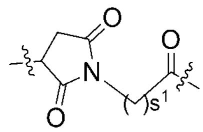,

and $s^1$ is an integer from 2 to 8;
$L^2$ is a tetrapeptide residue; preferably, $L^2$ is a tetrapeptide residue of GGFG;
$L^3$ is $-NR^3(CR^4R^5)_t$, wherein $R^3$, $R^4$, and $R^5$ are identical or different and are each independently a hydrogen atom or $C_{1-6}$ alkyl, and t is 1 or 2;
wherein the $L^1$ terminus is attached to Pc, and the $L^3$ terminus is attached to a drug.

**9.** The antibody-drug conjugate of general formula Pc-L-Y-D or the pharmaceutically acceptable salt thereof according to any one of claims 1 to 8, wherein the antibody-drug conjugate has a structure shown below:

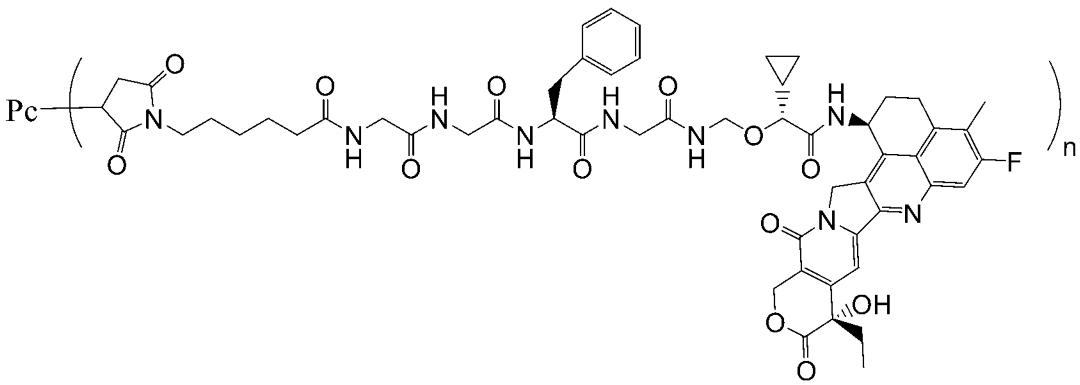

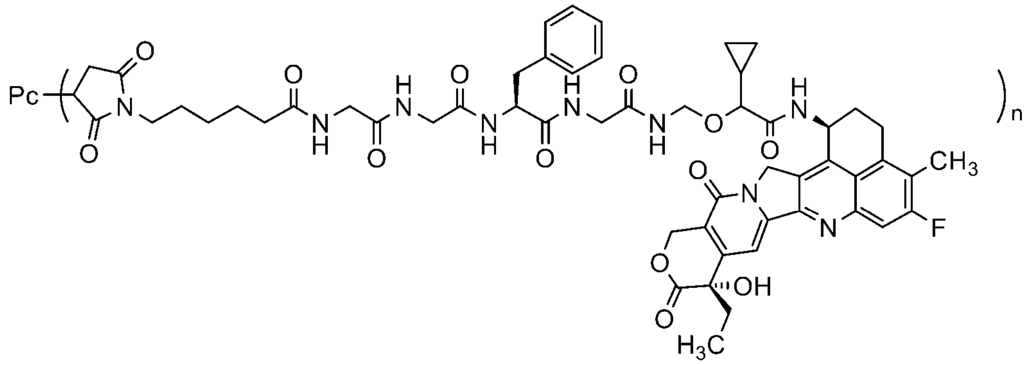

,

or

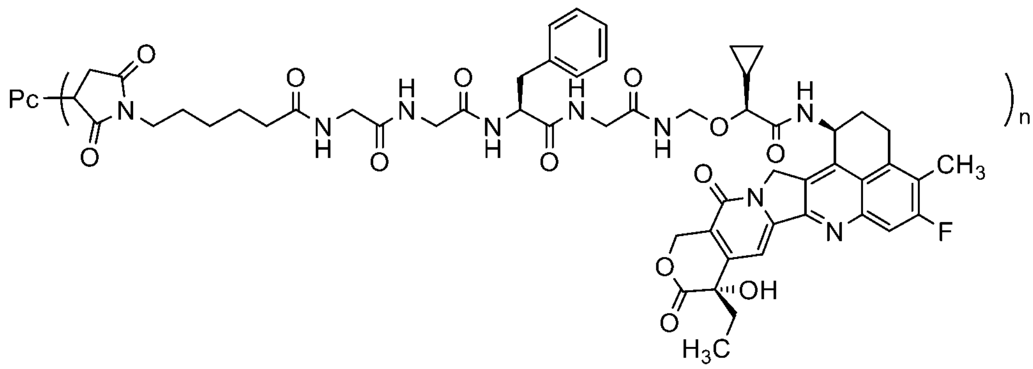

.

wherein
Pc is an anti-PSMA antibody or an antigen-binding fragment thereof, comprising a heavy chain variable region and a light chain variable region, wherein:

the heavy chain variable region comprises the HCDR1, the HCDR2, and the HCDR3 set forth in SEQ ID NO: 5, SEQ ID NO: 6, and SEQ ID NO: 7, respectively; the light chain variable region comprises the LCDR1, the LCDR2, and the LCDR3 set forth in SEQ ID NO: 8, SEQ ID NO: 9, and SEQ ID NO: 10, respectively;
n is a decimal or an integer from 1 to 10.

**10.** The antibody-drug conjugate of general formula Pc-L-Y-D or the pharmaceutically acceptable salt thereof according to any one of claims 1 to 9, wherein the antibody-drug conjugate is:

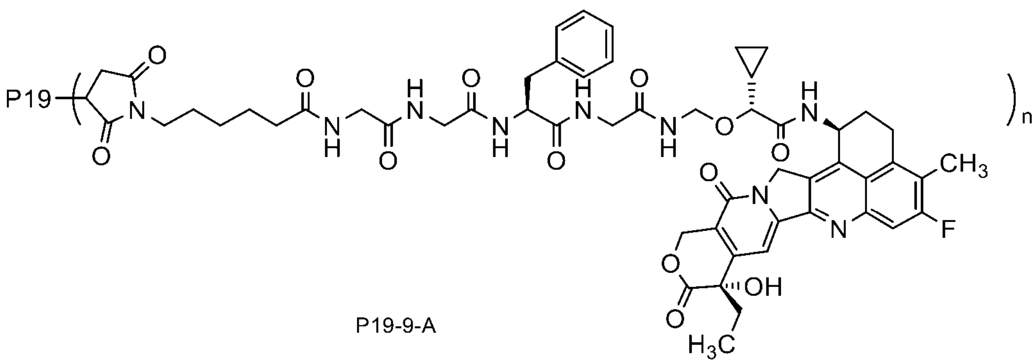

P19-9-A

wherein:

n is a decimal or an integer from 1 to 8, preferably from 3 to 8, and more preferably from 5 to 8;
P19 is an anti-PSMA antibody, comprising the heavy chain set forth in SEQ ID NO: 19 and the light chain set forth in SEQ ID NO: 20.

**11.** A pharmaceutical composition, comprising:

the antibody-drug conjugate of general formula Pc-L-Y-D or the pharmaceutically acceptable salt thereof according to any one of claims 1 to 10, and
one or more pharmaceutically acceptable excipients, diluents, or carriers.

**12.** Use of the antibody-drug conjugate of general formula Pc-L-Y-D or the pharmaceutically acceptable salt thereof according to any one of claims 1 to 10, or the pharmaceutical composition according to claim 11 in the manufacture of a medicament for treating a PSMA-mediated disease or disorder.

**13.** Use of the antibody-drug conjugate or the pharmaceutically acceptable salt thereof according to any one of claims 1 to 10, or the pharmaceutical composition according to claim 11 in the manufacture of a medicament for treating and/or preventing a tumor or cancer, wherein the tumor or cancer is preferably prostate cancer, head and neck squamous cell carcinoma, head and neck cancer, brain cancer, neuroglioma, glioblastoma multiforme, neuroblastoma, neuroendocrine neoplasm, throat cancer, nasopharyngeal carcinoma, esophageal cancer, thyroid cancer, malignant pleural mesothelioma, lung cancer, breast cancer, liver cancer, hepatobiliary cancer, pancreatic cancer, gastric cancer, colorectal cancer, renal cancer, ovarian cancer, endometrial cancer, cervical cancer, bladder cancer, testicular cancer, melanoma, leukemia, lymphoma, chondrosarcoma, multiple myeloma, myelodysplastic syndrome, Krukenberg tumor, squamous cell carcinoma, Ewing sarcoma, urothelial carcinoma, or Merkel cell carcinoma;

preferably, the lymphoma is selected from the group consisting of Hodgkin lymphoma, non-Hodgkin lymphoma, diffuse large B-cell lymphoma, follicular lymphoma, primary mediastinal large B-cell lymphoma, mantle cell lymphoma, small lymphocytic lymphoma, T-cell/histiocyte-rich large B-cell lymphoma, and lymphoplasmacytic lymphoma;
the lung cancer is selected from the group consisting of non-small cell lung cancer and
small cell lung cancer; the leukemia is selected from the group consisting of chronic myeloid leukemia, acute myeloid leukemia, lymphocytic leukemia, lymphoblastic leukemia, acute lymphoblastic leukemia, chronic lymphocytic leukemia, and myeloid cell leukemia.

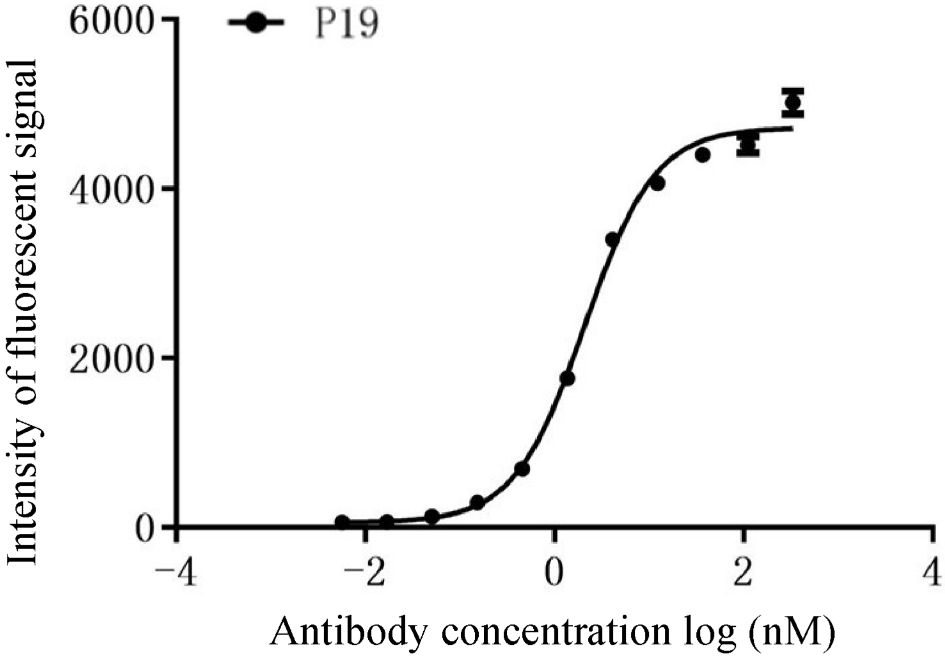
P19
6000
4000
2000
0
Intensity of fluorescent signal
−4
−2
0
2
4
Antibody concentration log (nM)

**FIG. 1A**

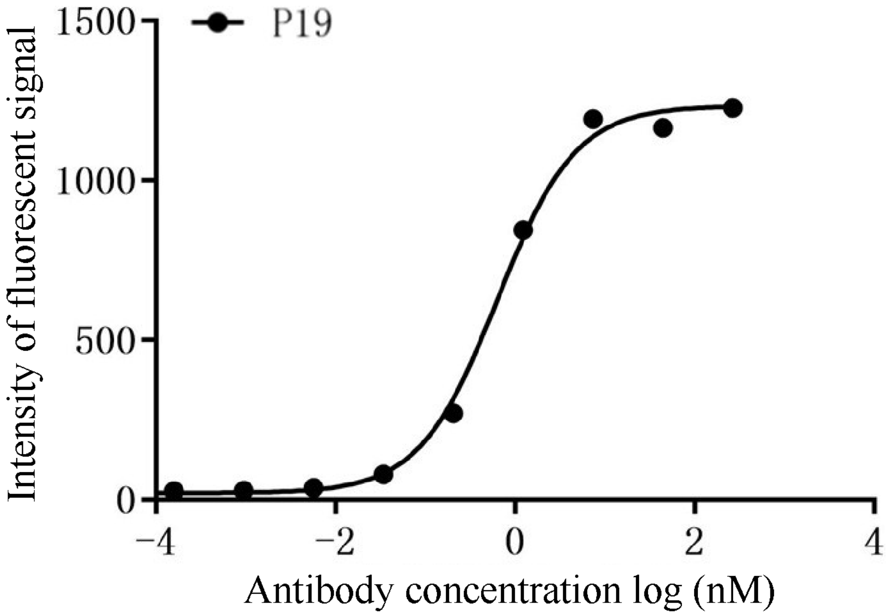
P19
1500
1000
500
0
Intensity of fluorescent signal
−4
−2
0
2
4
Antibody concentration log (nM)

**FIG. 1B**

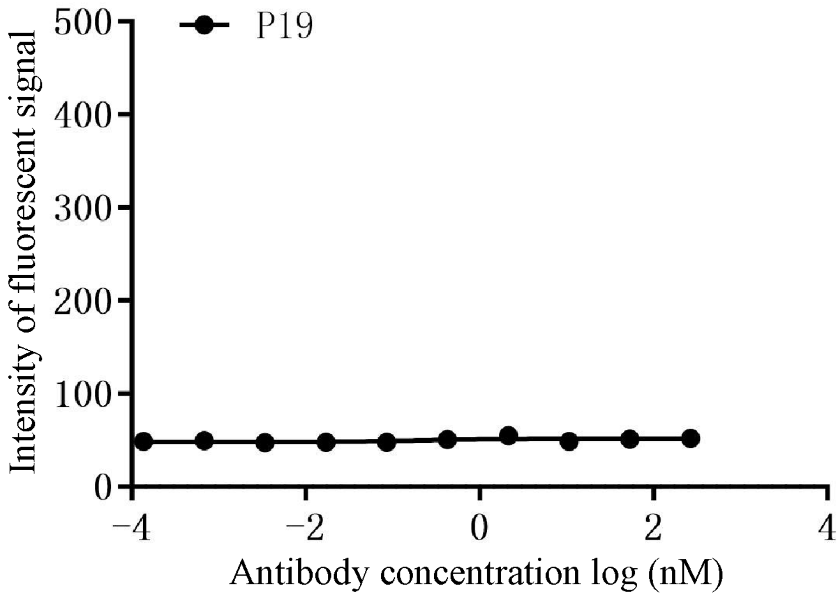

P19
Intensity of fluorescent signal
500
400
300
200
100
0
−4
−2
0
2
4
Antibody concentration log (nM)


FIG. 1C

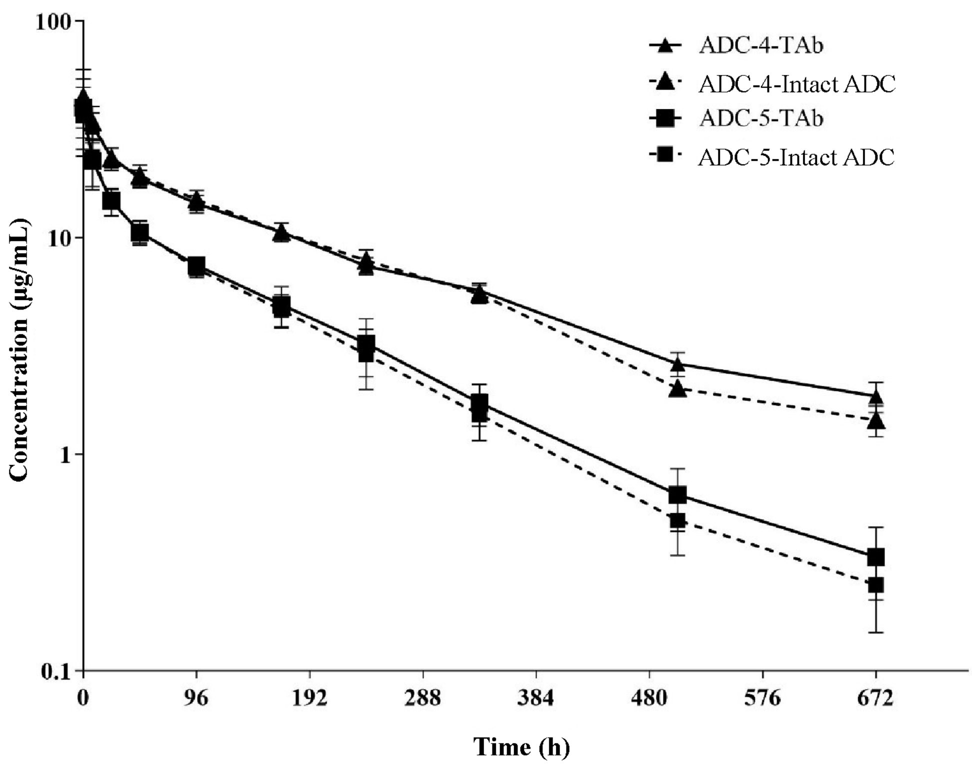

ADC-4-TAb
ADC-4-Intact ADC
ADC-5-TAb
ADC-5-Intact ADC
Concentration (µg/mL)
100
10
1
0.1
0
96
192
288
384
480
576
672
Time (h)


FIG. 2

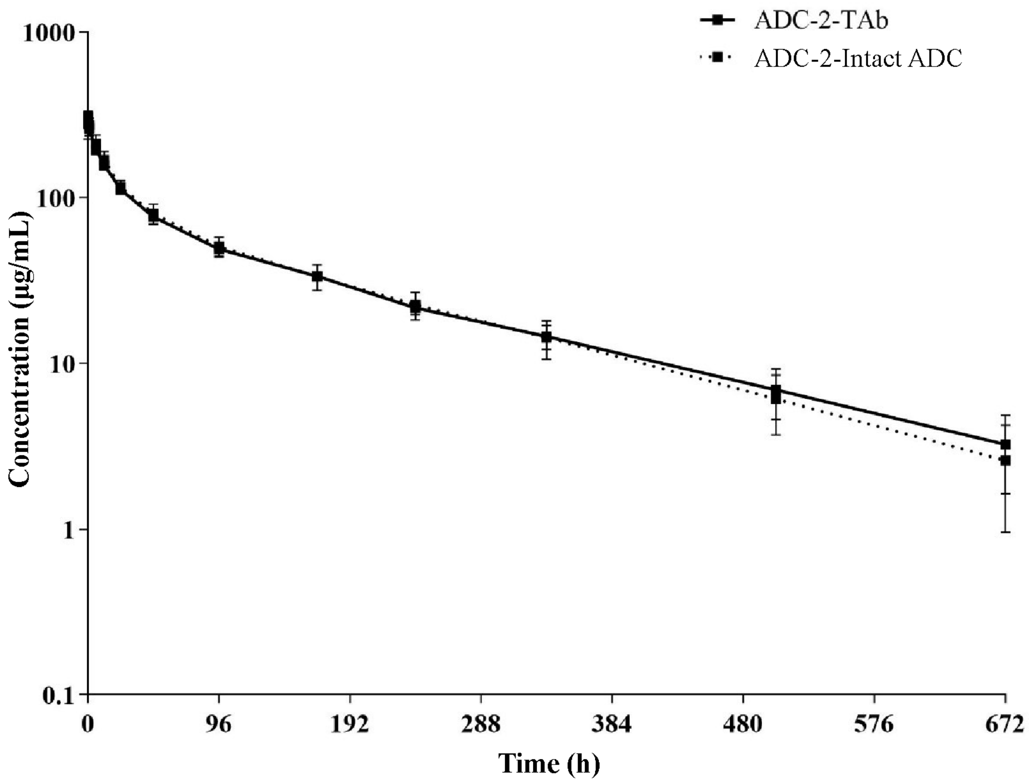
ADC-2-TAb
ADC-2-Intact ADC
1000
100
10
1
0.1
Concentration (μg/mL)
0
96
192
288
384
480
576
672
Time (h)

FIG. 3

**INTERNATIONAL SEARCH REPORT**

International application No.
**PCT/CN2024/109472**

**A. CLASSIFICATION OF SUBJECT MATTER**

A61K 47/68(2017.01)i; A61K 47/65(2017.01)i; A61K 31/506(2006.01)i; C07D 491/22(2006.01)i; A61P 35/00(2006.01)i; A61P 37/02(2006.01)i

According to International Patent Classification (IPC) or to both national classification and IPC

**B. FIELDS SEARCHED**

Minimum documentation searched (classification system followed by classification symbols)

IPC: A61K; C07D; A61P

Documentation searched other than minimum documentation to the extent that such documents are included in the fields searched

Electronic data base consulted during the international search (name of data base and, where practicable, search terms used)

CNABS, CNTXT, CNKI, VEN, ENTXT, TWTXT, ISI Web of Science, NCBI, BING, GENBANK, STNext, 中国专利生物序列检索系统, China Patents Biological Sequence Search System: 前列腺特异性膜抗原, 依喜替康, 艾沙替康, 依沙替康, 抗体, 偶联, 癌症, 肿瘤, 恒瑞, PSMA, prostate specific membrane antigen, DX 8951, exatecan, irinotecan, DX-8951a, conjugate, ADC, antibody, cancer, tumor, carcinoma, 171335-80-1, 序列检索, structure search

**C. DOCUMENTS CONSIDERED TO BE RELEVANT**

| Category* | Citation of document, with indication, where appropriate, of the relevant passages | Relevant to claim No. |
|---|---|---|
| PX | CN 116942845 A (JIANGSU HENGRUI MEDICINE CO., LTD. et al.) 27 October 2023 (2023-10-27) abstract, and claims 1-24 | 1-13 |
| PA | WO 2023147784 A1 (JIANGSU HENGRUI MEDICINE CO., LTD. et al.) 10 August 2023 (2023-08-10) abstract, and claims 1-18 | 1-13 |
| PA | WO 2023198015 A1 (JIANGSU HENGRUI MEDICINE CO., LTD. et al.) 19 October 2023 (2023-10-19) abstract, and claims 1-22 | 1-13 |
| A | WO 2021190583 A1 (JIANGSU HENGRUI MEDICINE CO., LTD. et al.) 30 September 2021 (2021-09-30) abstract, and claims 1-20 | 1-13 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 November 2024** | **11 November 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

INTERNATIONAL SEARCH REPORT

International application No.
PCT/CN2024/109472

C. DOCUMENTS CONSIDERED TO BE RELEVANT

| Category* | Citation of document, with indication, where appropriate, of the relevant passages | Relevant to claim No. |
|---|---|---|
| A | WO 2020063676 A1 (JIANGSU HENGRUI MEDICINE CO., LTD. et al.) 02 April 2020 (2020-04-02) abstract, and claims 1-46 | 1-13 |
| A | CN 111989138 A (AMBRX INC.) 24 November 2020 (2020-11-24) abstract, and claims 1-24 | 1-13 |
| A | CN 101287498 A (PSMA DEVELOPMENT COMPANY, LLC) 15 October 2008 (2008-10-15) abstract, and claims 1-120 | 1-13 |

Form PCT/ISA/210 (second sheet) (July 2022)

**INTERNATIONAL SEARCH REPORT**

International application No.
**PCT/CN2024/109472**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

**INTERNATIONAL SEARCH REPORT**
**Information on patent family members**

International application No.
**PCT/CN2024/109472**

| Patent document cited in search report | | | Publication date (day/month/year) | Patent family member(s) | | | Publication date (day/month/year) |
|---|---|---|---|---|---|---|---|
| CN | 116942845 | A | 27 October 2023 | None | | | |
| WO | 2023147784 | A1 | 10 August 2023 | TW | 202337902 | A | 01 October 2023 |
| | | | | KR | 20240139065 | A | 20 September 2024 |
| WO | 2023198015 | A1 | 19 October 2023 | TW | 202405015 | A | 01 February 2024 |
| WO | 2021190583 | A1 | 30 September 2021 | JP | 2023519261 | A | 10 May 2023 |
| | | | | TW | 202144016 | A | 01 December 2021 |
| | | | | EP | 4130006 | A1 | 08 February 2023 |
| | | | | EP | 4130006 | A4 | 17 January 2024 |
| | | | | BR | 112022019027 | A2 | 01 November 2022 |
| | | | | US | 2023140397 | A1 | 04 May 2023 |
| | | | | KR | 20220160016 | A | 05 December 2022 |
| | | | | CA | 3177279 | A1 | 30 September 2021 |
| | | | | ZA | 202210724 | B | 20 December 2023 |
| | | | | AU | 2021240756 | A1 | 17 November 2022 |
| | | | | MX | 2022011808 | A | 06 December 2022 |
| WO | 2020063676 | A1 | 02 April 2020 | ZA | 202102544 | B | 25 October 2023 |
| | | | | CA | 3114137 | A1 | 02 April 2020 |
| | | | | JP | 2022511351 | A | 31 January 2022 |
| | | | | JP | 7558156 | B2 | 30 September 2024 |
| | | | | BR | 112021004656 | A2 | 01 June 2021 |
| | | | | EP | 3858386 | A1 | 04 August 2021 |
| | | | | EP | 3858386 | A4 | 12 October 2022 |
| | | | | KR | 20210066833 | A | 07 June 2021 |
| | | | | MX | 2021003382 | A | 27 May 2021 |
| | | | | US | 2021353764 | A1 | 18 November 2021 |
| | | | | AU | 2019349207 | A1 | 08 April 2021 |
| | | | | TW | 202027795 | A | 01 August 2020 |
| | | | | TWI | 826543 | B | 21 December 2023 |
| CN | 111989138 | A | 24 November 2020 | JP | 2021519076 | A | 10 August 2021 |
| | | | | JP | 7512202 | B2 | 08 July 2024 |
| | | | | LT | 3773910 | T | 25 September 2024 |
| | | | | BR | 112020019611 | A2 | 05 January 2021 |
| | | | | US | 2021015940 | A1 | 21 January 2021 |
| | | | | US | 12059473 | B2 | 13 August 2024 |
| | | | | SG | 11202009670 | RA | 29 October 2020 |
| | | | | KR | 20200138759 | A | 10 December 2020 |
| | | | | CA | 3094985 | A1 | 03 October 2019 |
| | | | | DK | 3773910 | T3 | 19 August 2024 |
| | | | | WO | 2019191728 | A1 | 03 October 2019 |
| | | | | FI | 3773910 | T3 | 29 August 2024 |
| | | | | IL | 277357 | A | 30 November 2020 |
| | | | | JP | 2024123231 | A | 10 September 2024 |
| | | | | AU | 2019245444 | A1 | 01 October 2020 |
| | | | | EP | 3773910 | A1 | 17 February 2021 |
| | | | | EP | 3773910 | B1 | 24 July 2024 |
| | | | | MX | 2020010104 | A | 06 November 2020 |
| CN | 101287498 | A | 15 October 2008 | ES | 2440482 | T3 | 29 January 2014 |
| | | | | EP | 1912677 | A2 | 23 April 2008 |
| | | | | EP | 1912677 | B1 | 02 October 2013 |

Form PCT/ISA/210 (patent family annex) (July 2022)

**INTERNATIONAL SEARCH REPORT**
**Information on patent family members**

International application No.
**PCT/CN2024/109472**

| Patent document cited in search report | Publication date (day/month/year) | Patent family member(s) | Publication date (day/month/year) |
|---|---|---|---|
| | | KR 20140084242 A | 04 July 2014 |
| | | US 2021023094 A1 | 28 January 2021 |
| | | US 2007160617 A1 | 12 July 2007 |
| | | JP 2013100314 A | 23 May 2013 |
| | | JP 6063747 B2 | 18 January 2017 |
| | | PL 1912677 T3 | 31 March 2014 |
| | | DK 1912677 T3 | 13 January 2014 |
| | | NZ 565075 A | 27 May 2011 |
| | | KR 20080031296 A | 08 April 2008 |
| | | PT 1912677 E | 23 December 2013 |
| | | WO 2007002222 A2 | 04 January 2007 |
| | | WO 2007002222 A3 | 09 August 2007 |
| | | JP 2008546792 A | 25 December 2008 |
| | | HK 1116697 A1 | 02 January 2009 |
| | | KR 20150126978 A | 13 November 2015 |
| | | JP 2015187117 A | 29 October 2015 |
| | | AU 2006262231 A1 | 04 January 2007 |
| | | AU 2006262231 B2 | 24 January 2013 |
| | | CA 2612762 A1 | 04 January 2007 |
| | | CA 2612762 C | 10 December 2013 |
| | | BRPI 0612529 A2 | 23 November 2010 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310976626
- CN 202310990738
- CN 202311485462
- US 20050238649 A1
- US 5208020 A
- WO 2003034903 A2
- WO 2020063676 A1


### Non-patent literature cited in the description


- *CA CANCER J CLIN*, 2019, vol. 69, 7-34
- *European Urology*, vol. 66 (6), 1190-1193
- *Journal of Nuclear Medicine*, vol. 59 (2), 177-182
- *The EMBO Journal*, 2006, vol. 25, 1375-1384
- *Clinical Cancer Research*, January 1997, vol. 3, 81-85
- *Urologic Oncology: Seminars and Original Investigations*, vol. 1 (1), 18-28
- *Clin Cancer Res.*, 15 November 2010, vol. 16 (22), 5414-5423
- *J Nucl Med*, 2017, vol. 58, 81-84
- **KABAT et al.** Sequences of Proteins of Immunological Interest. 1991
- **MARTIN, ACR.** *Protein Sequence and Structure Analysis of Antibody Variable Domains [J].*, 2001
- **LEFRANC, M.P. et al.** *Dev. Comp. Immunol.*, 2003, vol. 27, 55-77
- *Front Immunol.*, 16 October 2018, vol. 9, 2278
- *Prot. Sci.*, 2000, vol. 9, 487-496
- **CHARI et al.** *Cancer Research*, 1992, vol. 52, 127-131
- **HERMANSON, G. T**. Bioconjugate Techniques. Academic Press, 1996, 56-136, 456-493
- **SAMBROOK et al.** Molecular Cloning: A Laboratory Manual. Cold Spring Harbor Laboratory Press
- **AUSUBEL et al.** Current Protocols in Molecular Biology. Greene Publishing Association, Wiley Interscience